(19) Europäisches Patentamt
European Patent Office
Office européen des brevets

(11) Veröffentlichungsnummer: **0 172 786**
**B1**

(12) **EUROPÄISCHE PATENTSCHRIFT**

(45) Veröffentlichungstag der Patentschrift: **30.01.91**

(21) Anmeldenummer: **85810282.5**

(22) Anmeldetag: **19.06.85**

(51) Int. Cl.[5]: **C 07 D 239/42,**
C 07 D 239/46, A 01 N 43/54

# (54) Pyrimidinderivate wirksam als Schädlingsbekämpfungsmittel.

(30) Priorität: **25.06.84 CH 3055/84**

(43) Veröffentlichungstag der Anmeldung:
**26.02.86 Patentblatt 86/09**

(45) Bekanntmachung des Hinweises auf die Patenterteilung:
**30.01.91 Patentblatt 91/05**

(84) Benannte Vertragsstaaten:
**AT BE CH DE FR GB IT LI LU NL SE**

(56) Entgegenhaltungen:
**EP-A-0 013 143**
**EP-A-0 126 254**
**EP-A-0 135 472**
**EP-A-0 139 613**
**DD-A- 151 404**
**GB-A-1 388 825**
**JP-A-56 065 804**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Hubele, Adolf, Dr.**
**Obere Egg 9**
**CH-4312 Magden (CH)**
Erfinder: **Zondler, Helmut, Dr.**
**Oberwilerstrasse 49**
**CH-4103 Bottmingen (CH)**
Erfinder: **Riebli, Peter**
**Bünten 17**
**CH-4446 Buckten (CH)**
Erfinder: **Eckhardt, Wolfgang, Dr.**
**Breslauerstrasse 14**
**D-7850 Lörrach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 172 786 B1

**Beschreibung**

Die vorliegende Erfindung betrifft neue N-(2-Nitrophenyl)-2-aminopyrimidin-Derivate der nachstehenden Formel I.

Die Erfindung betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von schädlichen Mikroorganismen, vorzugsweise pflanzenschädigenden Pilzen und Bakterien.

Die erfindungsgemässen Verbindungen entsprechen der allgemeinen Formel I:

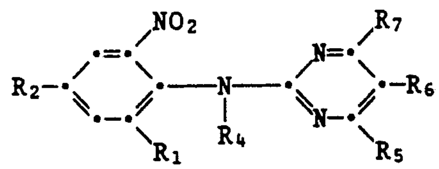

(I)

worin

$R_1$ und $R_2$ für $NO_2$ oder $CF_3$ stehen, mit der Massgabe, dass nur entweder $R_1$ oder $R_2$ für $NO_2$ stehen kann;

$R_4$ für Wasserstoff oder die Gruppe —C(O)R′ steht, wobei R′ unsubstituiertes oder durch Halogen oder $C_1$—$C_3$-Alkoxy substituiertes $C_1$—$C_4$-Alkyl bedeutet;

$R_5$ für Halogen, Cyano, $C_1$—$C_{12}$-Alkyl, $C_1$—$C_8$-Alkylthio, $C_1$—$C_6$-Alkylsulfonyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom unterbrochen sein kann, oder für die Gruppe $Q—(E)_n—(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E eine $C_1$—$C_3$-Alkylenbrücke bedeutet und X für Sauerstoff oder Schwefel steht;

$R_6$ für Halogen, Nitro, Cyano, $C_1$—$C_{12}$-Alkyl, $C_1$—$C_8$-Alkylthio, $C_1$—$C_6$-Alkylsulfonyl, $C_3$—$C_6$-Alkenyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom unterbrochen sein kann, oder für die Gruppe $Q—(E)_n—(X)_m$ steht, worin Q unsubstituiertes Phenyl darstellt und n und m 0 oder n 1 und m 0 oder n 0 und m 1 sind, oder worin Q ein durch Halogen, $C_1$—$C_3$-Alkyl, $C_1$—$C_3$-Alkoxy oder Nitro substituiertes Phenyl darstellt und n und m 0 sind; E eine $C_1$—$C_3$-Alkylenbrücke bedeutet und X für Sauerstoff oder Schwefel steht, und $R_6$ zusätzlich Cyclohexyloxy darstellt.

$R_7$ für Halogen, Rhodano, $C_1$—$C_{12}$-Alkyl, $C_1$—$C_8$-Alkylthio, $C_1$—$C_6$-Alkylsulfonyl, $C_1$—$C_6$-Alkylsulfoxyl, $C_2$—$C_5$-Alkenyl-$CH_2$-oxy, $C_2$—$C_5$-Alkinyl-$CH_2$-oxy, durch Halogen substituiertes $C_1$—$C_8$-Alkyl, unsubstituiertes oder durch Halogen substituiertes $C_1$—$C_8$-Alkoxy, dessen Alkylteil durch ein Sauerstoffatom unterbrochen sein kann, oder für die Gruppe $Q—(E)_n—(X)_m$ steht, worin Q unsubstituiertes Phenyl darstellt und n und m 0 oder n 0 und m 1 oder n und m 1 sind; E eine $C_1$—$C_3$-Alkylenbrücke bedeutet und X für Sauerstoff oder Schwefel steht; und

$R_6$ und/oder einer der Reste $R_5$ oder $R_7$ auch für Wasserstoff stehen können.

Unter dem Begriff Alkyl selbst oder als Bestandteil eines anderen Substituenten, wie Alkoxy, Haloalkyl, Haloalkoxy etc., sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl, Heptyl, Octyl, Nonyl, Decyl, Undecyl, Dodecyl usw.. sowie ihre Isomeren, wie z.B. Isopropyl, Isobutyl, tert.-Butyl, Isopentyl usw. Die Vorsilbe Halo in der Bezeichnung eines Substituenten bedeutet hier und im folgenden, dass dieser Substituent einfach bis perhalogeniert auftreten kann. Halogen und Halo stehen stellvertretend für Fluor, Chlor, Brom oder Jod. Haloalkyl steht somit für einen einfach bis perhalogenierten Alkylrest, wie z.B. $CHCl_2$, $CH_2F$, $CCl_3$, $CH_2Cl$, $CHF_2$, $CH_2CH_2Br$, $C_2Cl_5$, $CH_2Br$, CHBrCl usw., vorzugsweise für $CF_3$. Alkenyl steht z.B. für Allyl, Butenyl-(2) oder Butenyl-(3) sowie Ketten mit mehreren Doppelbindungen. Cycloalkyl steht je nach Zahl der genannten Kohlenstoffatome z.B. für Cyclopropyl, Cyclobutyl, Cyclopentyl, Cyclohexyl, Cycloheptyl, Cyclooctyl u.s.w. Alkinyl bedeutet z.B. Propinyl-(2), Propargyl, Butinyl-(2) usw., vorzugsweise Propargyl.

Unter einem gesättigten oder ungesättigten heterocyclischen Rest mit einem oder mehreren Heteroatomen soll hier und im folgenden vorzugsweise ein gesättigter oder ungesättigter fünf- oder sechsgliedriger Heterocyclus verstanden werden mit ein bis drei gleichen oder verschiedenen Heteroatomen, wie z.B. Sauerstoff, Stickstoff oder Schwefel. Typische Vertreter derartiger Heterocyclen sind: Tetrahydrofuran, Furan, Tetrahydrothiophen, Thiophen, Pyrrolidin, Pyrrol, Pyrrolin, Pyrazol, Imidazol, Pyrazolin, Oxazol, Thiazol, Isoxazol, Isothiazol, Pyran, Dihydropyran, Tetrahydropyran, Thiopyran, Dihydrothiopyran, Tetrahydrothiopyran, Pyridazin, Dihydropyridazin, Tetrahydropyridazin, Pyrimidin, Dihydropyrimidin, Tetrahydropyrimidin, Pyrazin, Dihydropyrazin, Tetrahydropyrazin, Morpholin, Thiazin, Dihydrothiazin, Tetrahydrothiazin, Piperazin und Triazin. $C_1$—$C_3$-Alkylen steht z.B. für folgende Gruppen: $—CH_2—$,

$CH_2CH_2$—, $CH_2CH_2CH_2$—, —$CH(CH_3)$—$CH_2$—, —$CH_2CH(CH_3)$—, —$CH(C_2H_5)$—, —$C(CH_3)_2$—. Alkylsulfoxy steht für die Gruppe Alkyl—S(O)—. $C_6H_5$ repräsentiert Phenyl. Die Verbindungen der Formel I sind bei Raumtemperatur Oele, Harze oder überwiegend kristalline Feststoffe, die sich durch sehr wertvolle biozide Eigenschaften auszeichnen. Sie lassen sich beispielsweise auf dem Agrarsektor oder verwandten Gebieten präventiv und kurativ zur Bekämpfung von phytopathogenen Schädlingen, wie z.B. Pilzen oder Bakterien, einsetzen. Die erfindungsgemässen Wirkstoffe der Formel zeichnen sich in weiten Anwendungskonzentrationen durch eine hohe biozide Aktivität und grosse Wirkungsbreite aus und können problemlos insbesondere im Agrarbereich eingesetzt werden.

Folgende Wirkstoffgruppen sind auf Grund ihrer ausgeprägten bioziden, insbesondere pflanzenfungiziden Aktivität bevorzugt:

*Gruppe Ia:* Verbindungen der Formel I, worin $R_1$, $R_2$ und $R_4$ die unter Formel I angegebenen Bedeutungen haben;

$R_5$ für Halogen, Cyano, $C_1$—$C_6$-Alkyl, $C_1$—$C_3$-Alkylthio, $C_1$—$C_3$-Alkylsulfonyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E für eine Methylenbrücke steht und X für Sauerstoff oder Schwefel bedeutet;

$R_6$ Halogen, Nitro, Cyano, $C_1$—$C_6$-Alkyl, $C_1$—$C_3$-Alkylthio, $C_1$—$C_3$-Alkylsulfonyl, $C_3$—$C_4$-Alkenyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, Methyl, Methoxy oder Nitro substituiertes Phenyl darstellt und n und m 0 sind; E für eine Methylenbrücke steht und X Sauerstoff oder Schwefel bedeutet;

$R_7$ für Halogen, $C_1$—$C_6$-Alkyl, $C_1$—$C_3$-Alkylthio, $C_1$—$C_3$-Alkylsulfonyl, $C_1$—$C_3$-Alkylsulfoxyl, durch Halogen substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 0 und m 1 oder n und m 1 sind; E für eine Methylenbrücke steht und X Sauerstoff oder Schwefel bedeutet; und

$R_6$ und/oder einer der Reste $R_5$ oder $R_7$ auch für Wasserstoff stehen können.

*Gruppe Ib:* Verbindungen der Formel I, worin $R_1$ und $R_2$ die unter Formel I angegebenen Bedeutungen haben;

$R_4$ Wasserstoff darstellt; und

$R_5$ für Halogen, Cyano, $C_1$—$C_6$-Alkyl, $C_1$—$C_3$-Alkylthio, $C_1$—$C_3$-Alkylsulfonyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E für eine Methylenbrücke steht und X Sauerstoff oder Schwefel bedeutet;

$R_6$ für Halogen, Nitro, Cyano, $C_1$—$C_6$-Alkyl, $C_1$—$C_3$-Alkylthio, $C_1$—$C_3$-Alkylsulfonyl, $C_3$—$C_4$-Alkenyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, Methyl, Methoxy oder Nitro substituiertes Phenyl darstellt und n und m 0 sind; E für eine Methylenbrücke steht und X Sauerstoff oder Schwefel bedeutet;

$R_7$ für Halogen, $C_1$—$C_6$-Alkyl, $C_1$—$C_3$-Alkylthio, $C_1$—$C_3$-Alkylsulfonyl, $C_1$—$C_3$-Alkylsulfoxyl, durch Halogen substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 0 und m 1 oder n und m 1 sind; E für eine Methylenbrücke steht und X Sauerstoff oder Schwefel bedeutet; und

$R_6$ und/oder einer der Reste $R_5$ oder $R_7$ auch für Wasserstoff stehen können.

*Gruppe Ic:* Verbindungen der Formel I, worin $R_1$ für $CF_3$ steht; $R_2$ für $NO_2$ steht; $R_4$ und $R_6$ Wasserstoff bedeuten; $R_5$ für Fluor, Chlor, Brom, $C_1$—$C_3$-Alkyl, $C_1$—$C_3$-Haloalkyl oder $C_1$—$C_3$-Alkoxy steht; und $R_7$ für Fluor, Chlor, Brom, $C_1$—$C_3$-Alkyl, $C_1$—$C_3$-Haloalkyl, $C_1$—$C_3$-Alkoxy oder $C_1$—$C_3$-Haloalkoxy steht.

Ferner besitzen folgende Verbindungen der Formel I, die sich durch spezielle Substitutionsmerkmale auszeichnen, besonders vorteilhafte fungizide Eigenschaften:

*Gruppe IIa:* Verbindungen der Formel I, worin $R_1$, $CF_3$ und $R_2$ $NO_2$ darstellen, $R_4$ für Wasserstoff steht und $R_5$, $R_6$ und $R_7$ mit Ausnahme von Wasserstoff die unter der Formel I angegebenen Bedeutungen besitzen.

*Gruppe IIb:* Verbindungen der Formel I, worin $R_1$ $CF_3$ und $R_2$ $NO_2$ darstellen, $R_4$ für Wasserstoff steht und $R_5$ Halogen, $C_1$—$C_6$-Alkyl, $C_1$—$C_8$-Alkoxy, $C_1$—$C_3$-Alkylthio, durch Halogen oder $C_1$—$C_4$-Alkoxy substituiertes $C_1$—$C_6$-Alkyl oder $C_1$—$C_4$-Alkoxy; $R_6$ Halogen, $C_1$—$C_6$-Alkyl, $C_1$—$C_8$-Alkoxy, $C_1$—$C_3$-Alkylthio,

$C_3$—$C_6$-Alkenyl, durch Halogen oder $C_1$—$C_4$-Alkoxy substituiertes $C_1$—$C_6$-Alkyl oder $C_1$—$C_4$-Alkoxy; und $R_7$ Halogen, $C_1$—$C_6$-Alkyl, $C_1$—$C_8$-Alkoxy, $C_1$—$C_3$-Alkylthio, $C_2$—$C_5$-Alkenyl-$CH_2$-oxy, $C_2$—$C_5$-Alkinyl-$CH_2$-oxy, durch Halogen substituiertes $C_1$—$C_6$-Alkyl oder durch Halogen substituiertes $C_1$—$C_4$-Alkoxy bedeuten.

Besonders bevorzugte Einzelsubstanzen sind z.B.:

N-(2'-Trifluormethyl-4',6'-dinitrophenyl)-2-amino-4-chlor-6-difluormethoxy-pyrimidin (1.1);
N-(2'-Trifluormethyl-4',6'-dinitrophenyl)-2-amino-4,6-dichlor-pyrimidin (1.2);
N-(2'-Trifluormethyl-4',6'-dinitrophenyl)-2-amino-4,5,6-trichlor-pyrimidin (1.69);
N-(2'-Trifluormethyl-4',6'-dinitrophenyl)-2-amino-4,5-dichlor-6-methoxy-pyrimidin (1.136);
N-(2'-Trifluormethyl-4',6'-dinitrophenyl)-2-amino-4-chlor-5-brom-6-propin(2)yloxy-pyrimidin (1.133);
N-(2'-Trifluormethyl-4',6'-dinitrophenyl)-2-amino-4-chlor-5-brom-6-propen(2)yloxy-pyrimidin (1.131);
N-(2'-Trifluormethyl-4',6'-dinitrophenyl)-2-amino-4,5-dichor-6-(2,2,2-trifluorethoxy)-pyrimidin (1.143).

Die Verbindungen der Formel I werden erfindungsgemäss dadurch hergestellt, dass man eine Verbindung der Formel II

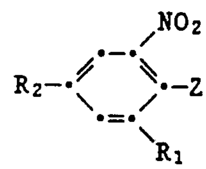

(II)

in Gegenwart einer Base mit einem Pyrimidin-Derivat der Formel III

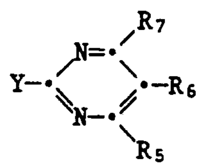

(III)

zu einer Verbindung der Formel I'

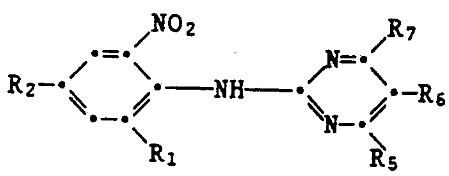

(I')

umsetzt, und letztere zur Herstellung N-acylierter Derivate mit einem reaktionsfähigen Derivat der Carbonsäure IV

$$R_4COOH \qquad (IV)$$

N-acyliert, wobei die Substituenten $R_1$ bis $R_7$ die unter Formel I angegebenen Bedeutungen haben und Z und Y für $NH_2$ oder Halogen stehen, wobei in dem Fall, in dem Z für Halogen steht, Y $NH_2$ bedeutet und in dem Fall in dem Z für $NH_2$ steht, Y Halogen bedeutet.

Für die Herstellung der Verbindungen der Formel I bzw. I' sind folgende Reaktionsbedingungen vorteilhaft:

Die N-Arylierung von (II) mit (III) zu (I'), sowie die N-Acylierung von (I') mit (IV) zu (I) erfolgen unter Halogenwasserstoffabspaltung. Die Reaktionstemperaturen liegen bei der N-Arylierung zwischen −20°C und 150°C, vorzugsweise −20°C und +30°C, bei der N-Acylierung zwischen 0° und +180°C, vorzugsweise 0° und 150°C bzw. am Siedepunkt des Lösungsmittels bzw. Lösungsmittelgemisches. In beiden Fällen ist die Verwendung von säurebindenden Mitteln bzw. Kondensationsmitteln vorteilhaft. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Alkoholate wie z.B. Kalium-tert.-butylat, Oxide und Hydroxide, Carbonate und Hydrogencarbonate von Alkali- und Erdalkalimetallen sowie Alkaliacetate.

Entstehender Halogenwasserstoff kann in manchen Fällen auch mittels Durchleiten von Inertgas, z.B. Stickstoff aus dem Reaktionsgemisch vertrieben werden.

Die Reaktionen können in Anwesenheit von reaktionsinerten Lösungs- oder Verdünnungsmitteln durchgeführt werden. In Frage kommen beispielsweise aliphatische und aromatische Kohlenwasserstoffe

wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann das Acylierungs- oder Arylierungsmittel selbst als Lösungsmittel dienen.

Die Reaktion (II) und (III) kann auch in einem wässrigen Zweiphasensystem nach dem allgemein bekannten Prinzip der Phasentransferkatalyse durchgeführt werden.

Für die organische, mit Wasser nicht mischbare Phase kommen dabei z.B. folgende Lösungsmittel in Frage: Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw. Beispiele geeigneter Phasentransfer-Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; tetrapropylammoniumchlorid, -bromid, jodid; usw.. Als Phasentransfer-Katalysatoren kommen auch Phosphonium-Salze in Betracht. Die Reaktionstemperaturen liegen im allgemeinen zwischen −30° und 130°C, bzw. am Siedepunkt des Lösungsmittels oder Lösungsmittelgemisches.

Im übrigen kann bei der Herstellung aller hierin genannten Ausgang-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid.

Das beschriebene Herstellungsverfahren ist, einschliesslich aller Teilschritte, ein wichtiger Bestandteil der vorliegenden Erfindung.

Phenyl-2-aminopyrimidin-Derivate sind als pestizid und auch als fungizid wirksam bereits bekannt. Sie können jedoch die in der Praxis an sie gestellten Forderungen nicht immer voll befriedigen. Beispielsweise sind derartige Verbindungen in der japanischen Offenlegungsschrift JP—A—56—65804 beschrieben. Sie haben die allgemeine Formel

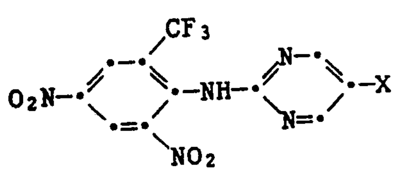

worin X Wasserstoff oder Halogen bedeutet. Vergleichsweise sind die erfindungsgemässen Wirkstoffe in ihrer fungiziden Aktivität diesen Verbindungen deutlich überlegen. Darüber hinaus sind weitere Derivate vom Phenylaminopyrimidin-Typ als Fungizide bekannt. Solche Verbindungen sind in folgenden Publikationen beschreiben: EP—135472, EP—126254, EP—139613 und DD—151404.

Bezüglich der erfindungsgemässen Verbindungen der Formel I wurde nun überraschend festgestellt, dass diese ein für praktische Bedürfnisse sehr günstiges biozides Spektrum gegen Pilze und Bakterien, insbesondere gegen phytopathogene Pilze und Bakterien aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen und Insekten verschont bleiben. Ferner können pflanzenschädigende Akarina und Nematoden sowie unerwünschte Pflanzenarten erfolgreich mit den Wirkstoffen der Formel I bekämpft werden.

Als Mikrobizide sind die Wirkstoffe der Formel I z.B. gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); insbesondere wirken sie gegen die Klasse der Ascomyceten (z.B. Venturia, Podosphaera, Erysiphe,

Monilinia, Uncinula). Sie können auch als Beizmittel zur Behandlung von Saatgut (Früchte, Knollen, Körner) und Pflanzenstecklingen zum Schutz vor Pilzinfektionen eingesetzt werden.

Die Erfindung betrifft somit auch Schädlingsbekämpfungsmittel insbesondere pflanzenfungizide Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für den hieren offenbarten mikrobiziden Einsatz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden überlicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selective Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte).

Die Verbindungen der Formel I können aber auch auf Samenkörper aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verbünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und dem gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der Physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge

führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen gewinnen kann. Verwendbare Handelsmischungen sind z.B. Phosphatidylcholin-Mischungen. Synthetische Phospholipide sind z.B. Dioctanoylphosphatidylcholin und Dipalmitoylphosphatidylcholin.

Als oberflächenaktive Verbindungen kommen je nach Art des zu formulierenden Wirkstoffes der Formel nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionishe Tenside können sowohl sog. wasserlösliche Seifen, als auch wasserlösliche synthetische oberflächenaktive Verbindungen sein.

Als Seifen seien die Alkali- Erdalkali- oder gegebenenfalls substituierten Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwedent, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8—22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 g Propylenglykolethergruppen enthaltenden Polyethylenoxidaddukte an Polypropylenglykol, Aethylendiaminopolypropylenglykol und Alkylpoly-propylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxylalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebraüchlichen Tenside sind u.a. in folgenden Publikationen beschrieben:

"McCutcheon's Detergents and Emulsifiers Annual" BC Publishing Corp., Ridgewood New Jersey, 1981; Helmut Stache "Tensid-Taschenbuch" Carl Hanser-Verlag München/Wien, 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbaucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzeilung spezieller Effekte enthalten.

Derartige agrochemische Mittel sind eine Bestandteil der vorliegenden Erfindung.

Die nachfolgenden Beispiele dienen zur näheren Erläuterung der Erfindung. Temperaturen sind in Celsiusgraden angegeben. Prozente und Teile beziehen sich auf das Gewicht.

1. Herstellungsbeispiele

Beispiel 1.1

Herstellung von

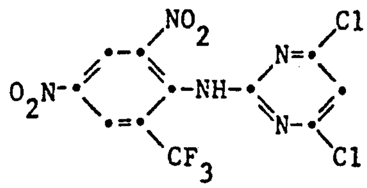

(Verb. Nr. 1.2)

N-(2'-Trifluormethyl-4',6'-dinitrophenyl)-2-amino-4,6-dichlor-pyrimidin

Eine Lösung von 15 Teilen 4,6-Dichlor-2-amino-pyrimidin in 400 ml Tetrahydrofuran wird bei Raumtemperatur unter Rühren portionsweise mit 13,2 Teilen 85%igen, pulverisiertem Kaliumhydroxid versetzt, wobei die Temperatur innerhalb einer halben Stunde auf 23° ansteigt. Das Reaktionsgemisch wird auf 5° abgekühlt, und zu der Suspension werden innerhalb von 1 Stunde 28 Teile 2-Chlor-3,5-dinitrobenzotrifluorid in 80 ml Tetrahydrofuran zugetropft, wobei sich die Suspension rot verfärbt. Nach 15 stündigem Rühren bei Raumtemperatur wird das Reaktionsgemisch auf Eiswasser gegossen, mit 10 ml konzentrierter Salzsäure angesäuert und zweimal mit je 200 ml Essigsäureethylester extrahiert. Die vereinigten Extrakte werden zweimal mit je 100 ml Wasser gewaschen, über Natriumsulfat getrocknet, filtriert und eingedampft. Die zurückbleibende Kristallmasse wird aus 400 ml Isopropanol umkristallisiert; gelbe Kristalle mit Smp. 170—173°C.

Analog den vorstehend beschriebenen Arbeitsweisen werden auch die nachfolgend aufgezählten Verbindungen der Formel I hergestellt:

Tabelle 1: Verbindungen der Formel

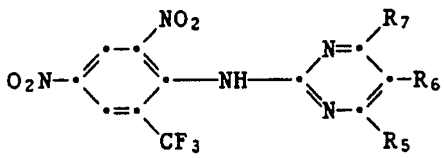

| Verb. Nr. | $R_5$ | $R_6$ | $R_7$ | physikalische Konstante [°C] |
|---|---|---|---|---|
| 1.1 | Cl | H | $OCHF_2$ | Smp. 159-161 |
| 1.2 | Cl | H | Cl | Smp. 170-173 |
| 1.3 | $SCH_3$ | CN | H | |
| 1.4 | $OCH_3$ | CN | H | |
| 1.5 | $CH_3$ | $CH_3$ | H | |
| 1.6 | $OCH_3$ | Br | H | |
| 1.7 | $OC_3H_7$-i | H | $OC_3H_7$-i | |
| 1.8 | $CH_3$ | CN | H | |
| 1.9 | $OC_2H_4OC_2H_5$ | H | $CH_3$ | |
| 1.10 | $SC_2H_5$ | H | $CH_3$ | |
| 1.11 | $OC_2H_5$ | H | F | |
| 1.12 | $OCH_3$ | $OC_2H_5$ | H | |
| 1.13 | $CH_3$ | J | $CH_3$ | Harz |
| 1.14 | $CH_3$ | CN | $S(O)CH_3$ | |
| 1.15 | $OC_2H_5$ | H | $CH_3$ | |
| 1.16 | $C_4H_9$-i | H | H | |
| 1.17 | Cl | $CH_3$ | H | |
| 1.18 | Cl | H | H | |

Tabelle 1: (Fortsetzung)

| Verb. Nr. | $R_5$ | $R_6$ | $R_7$ | physikalische Konstante [°C] |
|---|---|---|---|---|
| 1.19 | $C_6H_5$ | CN | H | |
| 1.20 | H | $CH_2-C_6H_5$ | H | |
| 1.21 | $CH_3$ | CN | $SO_2CH_3$ | |
| 1.22 | $C_3H_7$-n | H | H | |
| 1.23 | Cl | $OCH_3$ | H | |
| 1.24 | $CH_2OCH_3$ | H | $CH_3$ | |
| 1.25 | $CHBr_2$ | Br | H | |
| 1.26 | $OCH_3$ | $OCH_3$ | H | |
| 1.27 | Cl | H | $SC_2H_5$ | semikristallin |
| 1.28 | $CH_3$ | $NO_2$ | $SO_2CH_3$ | |
| 1.29 | $SC_3H_7$-i | H | $CH_3$ | |
| 1.30 | Cl | F | H | |
| 1.31 | $OC_2H_5$ | $OCH_3$ | H | |
| 1.32 | $CH_3$ | H | $SC_3H_7$-n | |
| 1.33 | $OCH_3$ | H | H | |
| 1.34 | $CH_3$ | $C_2H_4OC_2H_5$ | H | |
| 1.35 | Cl | H | $SO_2CH_3$ | Harz |
| 1.36 | $OC_2H_5$ | $NO_2$ | H | |
| 1.37 | $OCH_3$ | F | H | |
| 1.38 | $CH_3$ | H | $SCH_3$ | |
| 1.39 | $C_4H_9$-n | H | $CH_3$ | |
| 1.40 | $SC_2H_5$ | H | H | |

Tabelle 1: (Fortsetzung)

| Verb. Nr. | $R_5$ | $R_6$ | $R_7$ | physikalische Konstante [°C] |
|---|---|---|---|---|
| 1.41 | Cl | H | $OC_2H_5$ | Harz |
| 1.42 | Cl | $SO_2CH_3$ | H | |
| 1.43 | $C_3H_7$-i | H | H | |
| 1.44 | H | $C_4H_9$-n | H | |
| 1.45 | $CH_2OCH_3$ | $OCH_3$ | H | |
| 1.46 | H | CN | H | |
| 1.47 | Cl | $OC_2H_5$ | H | |
| 1.48 | $SO_2C_2H_5$ | H | H | |
| 1.49 | $CH_3$ | H | $O-C_6H_5$ | |
| 1.50 | $OCH_2-C_6H_5$ | H | F | |
| 1.51 | Cl | H | $SCH_3$ | Harz |
| 1.52 | F | H | F | |
| 1.53 | Cl | $CH_2-C_6H_5$ | $C_6H_5$ | |
| 1.54 | Br | H | H | |
| 1.55 | Cl | $SCH_3$ | H | |
| 1.56 | $CCl_3$ | H | $CCl_3$ | |
| 1.57 | H | $C_3H_7$-i | H | |
| 1.58 | $CH_3$ | H | $CF_3$ | |
| 1.59 | Cl | $S-C_6H_5$ | H | |
| 1.60 | $OC_3H_7$-i | H | $CH_3$ | |

Tabelle 1: (Fortsetzung)

| Verb. Nr. | $R_5$ | $R_6$ | $R_7$ | physikalische Konstante [°C] |
|---|---|---|---|---|
| 1.61 | Cl | H | SCN | semikristallin |
| 1.62 | $CH_2-C_6H_5$ | H | $CH_3$ | |
| 1.63 | Cl | $CH_2-C_6H_5$ | Cl | |
| 1.64 | $SCH_2-C_6H_5$ | H | $CH_3$ | |
| 1.65 | Cl | Br | Cl | Smp. 206-208 |
| 1.66 | Cl | $CH_2-C_6H_5$ | $CH_3$ | |
| 1.67 | Cl | H | $OCH_2F$ | Harz |
| 1.68 | Cl | $C_6H_5$ | Cl | |
| 1.69 | Cl | Cl | Cl | Smp. 177-178 |
| 1.70 | H | $CH_3$ | H | |
| 1.71 | $SCH_2-C_6H_5$ | H | $SCH_2-C_6H_5$ | |
| 1.72 | Cl | Br | Cl | |
| 1.73 | Cl | $CH_3$ | Cl | |
| 1.74 | H | $OC_4H_9$ | H | |
| 1.75 | Cl | $OC_2H_5$ | Cl | |
| 1.76 | Cl | Cl | $CH_3$ | |
| 1.77 | Cl | $C_5H_9$-n | $CH_3$ | |
| 1.78 | $SCH_3$ | H | $SCH_3$ | |
| 1.79 | Cl | $C_4H_9$-n | Cl | |
| 1.80 | H | $OC_4H_9$-sek. | H | |

Tabelle 1: (Fortsetzung)

| Verb. Nr. | $R_5$ | $R_6$ | $R_7$ | physikalische Konstante [°C] |
|---|---|---|---|---|
| 1.81 | H | $NO_2$ | H | |
| 1.82 | $CH_3$ | $C_6H_5$ | $CH_3$ | |
| 1.83 | $CH_3$ | $CH_3$ | $CH_3$ | |
| 1.84 | H | O-Cyclohexyl | H | |
| 1.85 | $SC_3H_7$-n | H | $SC_3H_7$-n | |
| 1.86 | H | $OC_2H_5$ | H | |
| 1.87 | Br | Cl | Cl | |
| 1.88 | Br | Cl | Br | |
| 1.89 | $OCH_3$ | $OCH_3$ | $OCH_3$ | |
| 1.90 | Cl | H | $OC(CH_3)_2C{\equiv}CH$ | |
| 1.91 | CN | H | $CH_3$ | |
| 1.92 | Cl | $NO_2$ | $OCH_3$ | |
| 1.93 | H | $OC_2H_4OCH_3$ | H | |
| 1.94 | Cl | H | $C_6H_5$ | |
| 1.95 | $CH_3$ | $CH_2$-$CH{=}CH_2$ | $CH_3$ | |
| 1.96 | $C_6H_5$ | H | $C_6H_5$ | |
| 1.97 | $SC_4H_9$-n | H | $CH_3$ | |
| 1.98 | $CH_3$ | H | $C_6H_5$ | |
| 1.99 | Br | H | Br | |
| 1.100 | $OC_2H_5$ | H | $OC_2H_5$ | |

Tabelle 1: (Fortsetzung)

| Verb. Nr. | $R_5$ | $R_6$ | $R_7$ | physikalische Konstante [°C] |
|---|---|---|---|---|
| 1.101 | H | $CH_2OCH_3$ | H | |
| 1.102 | Cl | $OCH_3$ | Cl | |
| 1.103 | $OCH_3$ | H | $SCH_3$ | |
| 1.104 | Cl | $OCH_3$ | $CH_3$ | |
| 1.105 | Cl | $C_3H_7$-n | $C_6H_5$ | |
| 1.106 | Cl | $C_6H_3Cl_2$(2,4) | Cl | |
| 1.107 | Cl | H | $SC_3H_7$-n | Harz |
| 1.108 | Cl | $C_6H_4(CH_3)$(4) | Cl | |
| 1.109 | Cl | Br | $CH_3$ | |
| 1.110 | Cl | $C_2H_5$ | Cl | |
| 1.111 | Cl | $C_6H_4(OCH_3)$(4) | Cl | |
| 1.112 | Cl | $CH_3$ | $C_6H_5$ | |
| 1.113 | $C_6H_4(CF_3)$(4) | CN | H | |
| 1.114 | Cl | $C_6H_4(NO_2)$(4) | Cl | |
| 1.115 | Cl | H | $SCH_3$ | Harz |
| 1.116 | Cl | H | $OCH_2$-Phenyl | |
| 1.117 | $C_6H_{13}$-n | H | H | |
| 1.118 | Cl | H | $OCH_2CH=CH_2$ | semikristallin |
| 1.119 | Cl | H | $OCH_2C\equiv CH$ | Smp. 130 |
| 1.120 | Cl | Br | $OCH_3$ | |
| 1.121 | Cl | Br | $OC_2H_5$ | |
| 1.122 | Cl | Br | $OC_3H_7$-i | |

Tabelle 1: (Fortsetzung)

| Verb. Nr. | $R_5$ | $R_6$ | $R_7$ | physikalische Konstante [°C] |
|---|---|---|---|---|
| 1.123 | Cl | Br | $OC_4H_9$-sek. | |
| 1.124 | Cl | Br | $OCH_2CF_3$ | |
| 1.125 | Cl | Br | $OCH_2CH_2Cl$ | |
| 1.126 | Cl | Br | $OCH_2CH_2Br$ | |
| 1.127 | Cl | Br | $OCHF_2$ | |
| 1.128 | Cl | Br | $OCH_2CH_2OCH_3$ | |
| 1.129 | Cl | Br | $SC_2H_5$ | |
| 1.130 | Cl | Br | $SC_3H_7$-i | |
| 1.131 | Cl | Br | $OCH_2CH{=}CH_2$ | Smp. 105-110 |
| 1.132 | Cl | Br | $OC(CH_3)_2C{\equiv}CH$ | |
| 1.133 | Cl | Br | $OCH_2C{\equiv}CH$ | Smp. 141-143 |
| 1.134 | Cl | Br | $OCH_2$-Phenyl | |
| 1.135 | Cl | Br | SCN | |
| 1.136 | Cl | Cl | $OCH_3$ | Smp. 168-169 |
| 1.137 | Cl | H | $OCH_3$ | Smp. 169-170 |
| 1.138 | Cl | Cl | $OCH_2CH{=}CH_2$ | Smp. 115-116 |
| 1.139 | Cl | H | $OCH_2CF_3$ | Smp. 139-140 |
| 1.140 | Cl | Cl | $SC_3H_7$-i | Smp. 146-148 |
| 1.141 | Cl | Cl | $OCH_2C{\equiv}CH$ | Smp. 134-135 |
| 1.142 | Cl | Cl | $OCH_2CH_2OCH_3$ | Smp. 102-105 |
| 1.143 | Cl | Cl | $OCH_2CF_3$ | Smp. 138-141 |
| 1.144 | H | $CF_3$ | H | |

Tabelle 2: Verbindungen der Formel

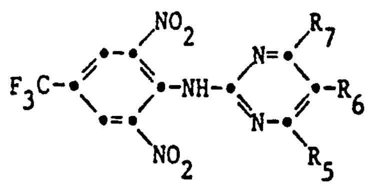

| Verb. Nr. | $R_5$ | $R_6$ | $R_7$ | physikalische Konstante [°C] |
|---|---|---|---|---|
| 2.1 | Cl | H | Cl | Smp. 144-145 |
| 2.2 | $CH_3$ | Cl | H | |
| 2.3 | $C_6H_{13}$-n | H | Cl | |
| 2.4 | Br | H | Br | |
| 2.5 | $CH_3$ | Cl | Cl | |
| 2.6 | Cl | $CH_2-C_6H_5$ | Cl | |
| 2.7 | Cl | Br | Cl | Smp. 193-195 |
| 2.8 | Cl | $OC_2H_5$ | Cl | |
| 2.9 | CN | H | $CH_3$ | |
| 2.10 | Cl | $C_4H_9$-n | Cl | |
| 2.11 | F | H | F | |
| 2.12 | H | CN | H | |
| 2.13 | $CH_3$ | $CH_3$ | H | |
| 2.14 | Cl | $C_6H_4Cl(4)$ | Cl | |
| 2.15 | $CH_3$ | Br | H | |
| 2.16 | Cl | $C_2H_5$ | Cl | |
| 2.17 | Cl | $C_6H_5$ | Cl | |

Tabelle 2 : (Fortsetzung)

| Verb. Nr. | $R_5$ | $R_6$ | $R_7$ | physikalische Konstante [°C] |
|---|---|---|---|---|
| 2.18 | Cl | $C_3H_7$-n | $C_6H_5$ | |
| 2.19 | Br | $C_6H_5$ | Br | |
| 2.20 | Cl | $CH_3$ | Cl | |
| 2.21 | $CH_3$ | CN | H | |
| 2.22 | Cl | $SCH_3$ | Cl | |
| 2.23 | Cl | $CH_2$-$C_6H_5$ | H | |
| 2.24 | Br | $C_3H_7$-n | Br | |
| 2.25 | $SCH_3$ | CN | H | |
| 2.26 | $OCH_3$ | CN | H | |
| 2.27 | $CH_3$ | H | $CH_3$ | Smp. 200-202 |
| 2.28 | Cl | H | $OCH_2CH{=}CH_2$ | Smp. 124 |
| 2.29 | Cl | H | $OCH_2C{\equiv}CH$ | Smp. 170-172 |
| 2.30 | Cl | H | $OC(CH_3)_2C{\equiv}CH$ | |
| 2.31 | Cl | H | $SCH_3$ | Smp. 153 |
| 2.32 | Cl | Cl | $OCH_3$ | Smp. 141-142 |
| 2.33 | Cl | Cl | $OC_2H_5$ | |
| 2.34 | Cl | Cl | $OC_3H_7$-i | |
| 2.35 | Cl | Cl | $OC_4H_9$-sek. | |
| 2.36 | Cl | Cl | $OC(CH_3)_3$ | |
| 2.37 | Cl | Cl | $OCH_2CF_3$ | Smp. 110-111 |
| 2.38 | Cl | Cl | $OCHF_2$ | |

Tabelle 2: (Fortsetzung)

| Verb. Nr. | $R_5$ | $R_6$ | $R_7$ | physikalische Konstante [°C] |
|---|---|---|---|---|
| 2.39 | Cl | Cl | $OCH_2CH_2Cl$ | |
| 2.40 | Cl | Cl | $OCH_2CH_2OC_2H_5$ | |
| 2.41 | Cl | Cl | $OCH_2CH=CH_2$ | Smp. 140-141 |
| 2.42 | Cl | Cl | $OCH_2C{\equiv}CH$ | Smp. 129-131 |
| 2.43 | Cl | Cl | $OC(CH_3)_2C{\equiv}CH$ | |
| 2.44 | Cl | Cl | $OCH_2$-Phenyl | |
| 2.45 | Cl | Cl | SCN | |
| 2.46 | Cl | Cl | $SC_2H_5$ | |
| 2.47 | Cl | Cl | $SC_3H_7$-i | $n_D^{23}$ 1,6188 |
| 2.48 | Cl | H | $OCH_3$ | Smp. 129-131 |
| 2.49 | Cl | Cl | Cl | Smp. 156-157 |
| 2.50 | Cl | Cl | $OCH_2CH_2OCH_3$ | Smp. 116-117 |
| 2.51 | Cl | Br | $OCH_2CH=CH_2$ | Smp. 120-122 |
| 2.52 | Cl | H | $OCH_2CF_3$ | Smp. 125-126 |
| 2.53 | Cl | Br | $OCH_2C{\equiv}CH$ | Smp. 143-144 |
| 2.54 | Cl | H | $CH_2Cl$ | Smp. 40-48 |
| 2.55 | Cl | H | $CH_2F$ | Smp. 108-112 |

<u>Tabelle 3:</u> Verbindungen der Formel

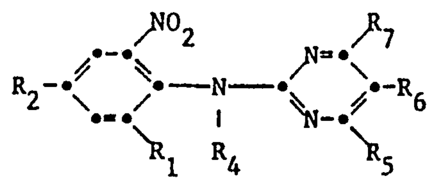

| Verb. Nr. | $R_1$ | $R_2$ | $R_4$ | $R_5$ | $R_6$ | $R_7$ | physikalische Konstante [°C] |
|---|---|---|---|---|---|---|---|
| 3.1 | $NO_2$ | $CF_3$ | $C(O)CH_3$ | Cl | H | Cl | Smp. 92-94 |
| 3.2 | $CF_3$ | $NO_2$ | $C(O)CH_3$ | Cl | H | Cl | |
| 3.3 | $NO_2$ | $CF_3$ | $C(O)CH_2Cl$ | Cl | $C_4H_9$-n | Cl | |
| 3.4 | $NO_2$ | $CF_3$ | $C(O)CH_2OCH_3$ | Cl | $CH_3$ | Cl | |
| 3.5 | $NO_2$ | $CF_3$ | $C(O)CH_3$ | Cl | $OC_2H_5$ | Cl | |
| 3.6 | $CF_3$ | $NO_2$ | $C(O)CH_2OC_2H_5$ | Cl | $CH_3$ | Cl | |
| 3.7 | $CF_3$ | $NO_2$ | $C(O)CH_2OCH_3$ | Cl | CN | $CH_3$ | |
| 3.8 | $NO_2$ | $CF_3$ | $C(O)CH_3$ | $SCH_3$ | CN | H | |
| 3.9 | $NO_2$ | $CF_3$ | $C(O)CH_3$ | $OCH_3$ | CN | H | |

2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | — | — |
| Tributylphenoyl-polyethylenglykol-ether (30 Mol Ethylenoxid) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen | 80% | 10% | 5% | 95% |
| Ethylenglykol-monomethyl-ether | 20% | — | — | — |
| Polyethylglykol M G 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 169—190°C) | — | — | 94% | — |

(MG = Molekulargewicht)

Die Lösungen sind zur Anwendung in Form kleinster Tropfen geeignet.

| 2.3 Granulate | a) | b) |
|---|---|---|
| Wirkstoff aus den Tabellen | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen | 2% | 5% |
| Hochdisperse Keiselsäure | 1% | 5% |
| Talkum | 97% | — |
| Kaolin | — | 90% |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| 2.5 Spritzpulver | a) | b) | c) |
|---|---|---|---|
| Wirkstoff aus den Tabellen | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyethylenglykolether (7—8 Mol Ethylenoxid) | — | 2% | — |
| Hochdisperse Kieselsäure | 5% | 10% | 10% |
| Kaolin | 62% | 27% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen | 10% |
| Octylphenolpolyethylenglykolether (4—5 Mol Ethylenoxid) | 3% |
| Ca-Dodecylbenzolsulfonat | 3% |
| Ricinusölpolyglykolether (35 Mol Ethylenoxid) | 4% |
| Cylohexanon | 30% |
| Xylolgemisch | 50% |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen | 5% | 8% |
| Talkum | 95% | — |
| Kaolin | — | 92% |

Man erhält anwendungsfertige Stäubemittel, indem der Wirkstoff mit den Träger vermischt auf einer geeigneten Mühle vermahlen wird.

| 2.8 Extruder Granulat | |
|---|---|
| Wirkstoff aus den Tabellen | 10% |
| N-Ligninsulfonat | 2% |
| Carboxymethylcellulose | 1% |
| Kaolin | 87% |

Der Wirkstoff wird mit den Zusatzstoffen vermischt, vermahlen und mit Wasser aufgefeuchtet. Dieses Gemisch wird extrudiert und anschliessend im Luftstrum getrocknet.

| 2.9 Umhüllungs-Granulat | |
|---|---|
| Wirkstoff aus den Tabellen | 3% |
| Polyethylenglykol (M G 200) | 3% |
| Kaolin | 94% |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man straubfreie Umhüllungs-Granulate.

| 2.10 Suspensions-Konzentrat | |
|---|---|
| Wirkstoff aus den Tabellen | 40% |
| Ethylenglykol | 10% |
| Nonylphenolpolyethylenglykolether (15 Mol Ethylenoxid) | 6% |
| N-Ligninsulfonat | 10% |
| Carboxymethylcelluose | 1% |
| 37%ige wässrige Formaldehyd-Lösung | 0,2% |
| Silikonöl in Former einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel 3.1

Residual-protektive Wirkung gegen Puccinia graminis auf Weizen

Weizenpflanzen wurden 6 Tage nach der Aussaat mit einer aus Spritzpulver des Wirkstoffes hergestellten Spritzbrühe (0,02% Aktivsubstanz) besprüht. Nach 24 Stunden wurden die behandelten Pflanzen mit einer Uredosporensuspension des Pilzes infiziert. Nach einer Inkubation während 48 Stunden bei 95—100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Verbindungen aus der Tabelle zeigten gegen Puccinia-Pilze eine sehr gute Wirkung. Unbehandelte aber infizierte Kontrollpflanzen zeigten einen Puccinia-Befall von 100%. Unter anderen hemmten die Verbindungen 1,1, 1,2 1.137, 1.139 und 1.143 den Puccinia-Befall auf 0 bis 5%.

Beispiel 3.2

Residual-protektive Wirkung gegen Cercopora arachidicola auf Erdnusspflanzen

10—15 cm hohe Erdnusspflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus den Tabellen behandelt wurden, einen stark reduzierten Cercospora-Befall. So verhinderten die Verbindungen 1.1, 1.2, 1.69, 1.133, 1.136, 1.139, 1.141 und 1.143 in obigen Versuchen das Auftreten von Flecken fast vollständig (0—10%).

Beispiel 3.3

Residual-protektive Wirkung gegen Erysiphae graminis auf Gerste

Ca. 8 cm hohe Gerstenpflanzen wurden mit einer aus Spritzpulver des Wirkstoffes hergestellten Spritzbrühe (0,002% Aktivsubstanz) besprüht. Nach 3—4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten gute Wirkung gegen Erysiphe-Pilze. Unbehandelte, aber infizierte Kontrollpflanzen zeigten einen Erysiphe-Befall von 100%. Verbindungen aus den Tabelle 1 und 2 hemmten den Pilzbefall bei Gerste auf weniger als 30%.

Beispiel 3.4

Residual-protektive Wirkung gegen Venturia inaequalis auf Apfeltrieben

Apfelstrecklinge mit 10—20 cm langen, Frischtrieben wurden mit einer aus Spritzpulver des Wirkstoffes hergestellten Spritzbrühe (0,02% Aktivsubstanz) besprüht. Nach 24 Stunden wurden die behandelten Pflanzen mit einer Konidiensuspension des Pilzes infiziert. Die Pflanzen wurden dann während 5 Tagen bei 90—100% relativer Luftfeuchtigkeit inkubiert und während 10 weiteren Tagen in einem Gewächshaus bei 20—24°C aufgestellt. Der Schorbefall wurde 15 Tage nach der Infektion beurteilt. Die Verbindungen 1.1, 1.2, 1.69, 1.311, 1.133, 1.139, 1.141, 1.142 und 1.143 hemmten den Krankheitsbefall auf 0—10%. Unbehandelte aber infizierte Kontrolltriebe wurden dagegen 100%ig befallen.

Beispiel 3.5

Residual-protektive Wirkung gegen Botrytis cinerea auf Bohnen

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95—100% relativer Luftfeuchtigkeit und 21°C erfolgte die Beurteilung des Pilzbefalls. Die Verbindungen aus der Tabelle hemmten in vielen Fällen die Pilzinfektion sehr stark. Bei einer Konzentration von 0,02% erwiesen sich z.B. die Verbindungen 1.1, 1.2, 1.69, 1.131, 1.133, 1.136, 1.138, 1.139 und 1.143 als voll wirksam. Der Krankheitsbefall lag bei 0 bis 10%. Der Bortrytis-Befall unbehandelter aber infizierter Bohnenpflanzen betrug 100%.

Beispiel 3.6

Residual-protektive Wirkung gegen Phytophtora infestans auf Tomatenpflanzen

Tomatenpflanzen wurden nach 3-wöchiger Anzucht mit einer aus Spritzpulver des Wirkstoffes hergestellten Spritzbrühe (0,02% Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90—100% relativer Luftfeuchtigkeit und 20°C.

Im obigen Versuch zeigten die Verbindungen Nr. 1.1, 1.2, 1.65, 1.69, 1.119, 1.131, 1.133, 1.136, 1.138, 1.140, 1.141, 1.142, und 1.143 eine sehr gute Wirkung. Im Gegensatz zu unbehandelten Kontrollpflanzen (mit 100% Befall) bewirkten diese Verbindungen eine fast vollständige Unterdrückung des Pilzbefalls (0 bis 10%).

Beispiel 3.7

Residual-protektive Wirkung gegen Plasmapora viticola auf Reben

Im 4—5 Blattstadium wurden Rebensämlinge mit einer aus Spritzpulver des Wirkstoffes hergestellten Spritzbrühe (0,02% Aktivsubstanz) besprüht. Nach 24 Stunden wurden die behandelten Pflanzen mit einer Sporangiensuspension des Pilzes infiziert. Nach einer Inkubation während 6 Tagen bei 95—100% relativer Luftfeuchtigkeit und 20°C wurde der Pilzbefall beurteilt.

Verbindungen aus den Tabellen zeigten gegen Plasmopara viticola auf Reben eine sehr gute fungizide Wirkung, insbesondere die Wirkstoffe Nr. 1.1, 1.2, 1.69, 1.131, 1.133, 1.136, 1.138, 1.139, 1.141, 1.142, 1.143, 2.52 und 2.53 bewirkten eine vollständige Unterdrückung des Pilzbefalls (0 bis 5%).

Beispiel 3.8

Residual-protektive Wirkung gegen Pyricularia oryzae auf Reispflanzen

Reispflanzen wurden nach zweiwöchiger Anzucht mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95—100% relativer Luftfeuchtigkeit und 24°C wurde der Pilzbefall beurteilt.

Reispflanzen, die mit einer Spritzbrühe behandelt worden sind, die als Aktivsubstanz eine der Verbindungen 1.143 oder 2.54 enthielt, zeigten im Vergleich zu unbehandelten Kontrollpflanzen (mit 100% Befall) weniger als 10% Pilzbefall.

**Patentansprüche für die Vertragsstaaten: BE CH DE FR GB IT LI LU NL SE**

1. Verbindungen der allgemeinen Formel I

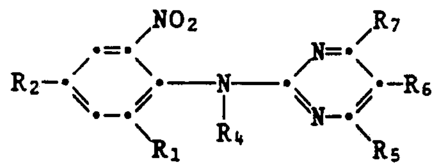

(I)

worin

$R_1$ und $R_2$ für $NO_2$ oder $CF_3$ stehen, mit der Massgabe, dass nur entweder $R_1$ oder $R_2$ für $NO_2$ stehen kann;

$R_4$ für Wasserstoff oder die Gruppe —C(O)R′ steht, wobei R′ unsubstituiertes oder durch Halogen oder $C_1$—$C_3$-Alkoxy substituiertes $C_1$—$C_4$-Alkyl bedeutet;

$R_5$ für Halogen, Cyano, $C_1$—$C_{12}$-Alkyl, $C_1$—$C_8$-Alkylthio, $C_1$—$C_6$-Alkylsulfonyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E eine $C_1$—$C_3$-Alkylenbrücke bedeutet und X für Sauerstoff oder Schwefel steht;

$R_6$ für Halogen, Nitro, Cyano, $C_1$—$C_{12}$-Alkyl, $C_1$—$C_8$-Alkylthio, $C_1$—$C_6$-Alkylsulfonyl, $C_3$—$C_6$-Alkenyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, $C_1$—$C_3$-Alkyl, $C_1$—$C_3$-Alkoxy der Nitro substituiertes Phenyl darstellt und n und m 0 sind; E eine $C_1$—$C_3$-Alkylenbrücke bedeutet und X für Sauerstoff oder Schwefel steht, und $R_6$ zusätzlich Cyclohexyloxy darstellt.

$R_7$ für Halogen, Rhodano, $C_1$—$C_{12}$-Alkyl, $C_1$—$C_8$-Alkylthio, $C_1$—$C_6$-Alkylsulfonyl, $C_1$—$C_6$-Alkylsulfoxyl, $C_2$—$C_5$-Alkenyl-$CH_2$-oxy, $C_2$—$C_5$-Alkinyl-$CH_2$-oxy, durch Halogen substituiertes $C_1$—$C_8$-Alkyl, unsubstituiertes oder durch Halogen substituiertes $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 0 und m 1 oder n und m 1 sind; E eine $C_1$—$C_3$-Alkylenbrücke bedeutet und X für Sauerstoff oder Schwefel steht; und

$R_6$ und/oder einer der Reste $R_5$ oder $R_7$ auch für Wasserstoff stehen können.

2. Verbindungen der Formel I nach Anspruch 1, dadurch gekennzeichnet, dass $R_1$, $R_2$ und $R_4$ die unter Formel I angegebenen Bedeutungen haben;

$R_5$ für Halogen, Cyano, $C_1$—$C_6$-Alkyl, $C_1$—$C_3$-Alkylthio, $C_1$—$C_3$-Alkylsulfonyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E für eine Methylenbrücke steht und X für Sauerstoff oder Schwefel bedeutet;

$R_6$ für Halogen, Nitro, Cyano, $C_1$—$C_6$-Alkyl, $C_1$—$C_3$-Alkylthio, $C_1$—$C_3$-Alkylsulfonyl, $C_3$—$C_4$-Alkenyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, Methyl, Methoxy oder Nitro substituiertes Phenyl darstellt und n und m 0 sind; E für eine Methylenbrücke steht und X Sauerstoff oder Schwefel bedeutet;

$R_7$ für Halogen, $C_1$—$C_6$-Alkyl, $C_1$—$C_3$-Alkylthio, $C_1$—$C_3$-Akylsulfonyl, $C_1$—$C_3$-Alkylsulfoxyl, durch Halogen substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 0 und m 1 oder n und m 1 sind; E für eine Methylenbrücke steht und X Sauerstoff oder Schwefel bedeutet; und

$R_6$ und/oder einer der Reste $R_5$ oder $R_7$ auch für Wasserstoff stehen können.

3. Verbindungen der Formel I nach Anspruch 2, dadurch gekennzeichnet, dass $R_1$ und $R_2$ die unter Formel I angegebenen Bedeutungen haben;

$R_4$ Wasserstoff darstellt; und

$R_5$ für Halogen, Cyano, $C_1$—$C_6$-Alkyl, $C_1$—$C_3$-Alkylthio, $C_1$—$C_3$-Alkylsulfonyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E für eine Methylenbrücke steht und X Sauerstoff oder Schwefel bedeutet;

$R_6$ für Halogen, Nitro, Cyano, $C_1$—$C_6$-Alkyl, $C_1$—$C_3$-Alkylthio, $C_1$—$C_3$-Alkylsulfonyl, $C_3$—$C_4$-Alkenyl,

durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, Methyl, Methoxy oder Nitro substituiertes Phenyl darstellt und n und m 0 sind; E für eine Methylenbrücke steht und X Sauerstoff oder Schwefel bedeutet;

$R_7$ für Halogen, $C_1$—$C_6$-Alkyl, $C_1$—$C_3$-Alkylthio, $C_1$—$C_3$-Alkylsulfonyl, $C_1$—$C_3$-Alkylsulfoxyl, durch Halogen substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt un n und m 0 oder n 0 oder m 1 oder n und m 1 sind; E für eine Methylenbrücke steht und X Sauerstoff oder Schwefel bedeutet; und

$R_6$ und/oder einer der Reste $R_5$ oder $R_7$ auch für Wasserstoff stehen können.

4. Verbindungen der Formel I nach Anspruch 1, dadurch gekennzeichnet, dass $R_1$ für $CF_3$ steht; $R_2$ für $NO_2$ steht; $R_4$ und $R_6$ Wasserstoff bedeuten; $R_5$ für Fluor, Chlor, Brom, $C_1$—$C_3$-Alkyl, $C_1$—$C_3$-Haloalkyl oder $C_1$—$C_3$-Alkoxy steht; und $R_7$ für Fluor, Chlor, Brom, $C_1$—$C_3$-Alkyl, $C_1$—$C_3$-Haloalkyl, $C_1$—$C_3$-Alkoxy oder $C_1$—$C_3$-Haloalkoxy steht.

5. Verbindungen der Formel I nach Anspruch 1, dadurch gekennzeichnet, dass $R_1$ $CF_3$ und $R_2$ $NO_2$ darstellen; für Wasserstoff steht;

$R_5$ für Halogen, Cyano, $C_1$—$C_{12}$-Alkyl, $C_1$—$C_8$-Alkylthio, $C_1$—$C_6$-Alkylsulfonyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E eine $C_1$—$C_3$-Alkylenbrücke bedeutet und X für Sauerstoff oder Schwefel steht;

$R_6$ für Halogen, Nitro, Cyano, $C_1$—$C_{12}$-Alkyl, $C_1$—$C_8$-Alkylthio, $C_1$—$C_6$-Alkylsulfonyl, $C_3$—$C_6$-Alkenyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, $C_1$—$C_3$-Alkyl, $C_1$—$C_3$-Alkoxy oder Nitro substituiertes Phenyl darstellt und n und m 0 sind; E eine $C_1$—$C_3$-Alkylenbrücke bedeutet und X für Sauerstoff oder Schwefel steht, und $R_6$ zusätzlich Cyclohexyloxy darstellt.

$R_7$ für Halogen, Rhodano, $C_1$—$C_{12}$-Alkyl, $C_1$—$C_8$-Alkylthio, $C_1$—$C_6$-Alkylsulfonyl, $C_1$—$C_6$-Alkylsulfoxyl, $C_2$—$C_5$-Alkenyl-$CH_2$-oxy, $C_2$—$C_5$-Alkinyl-$CH_2$-oxy, durch Halogen substituiertes $C_1$—$C_8$-Alkyl, unsubstituiertes oder durch Halogen substituiertes $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 0 und m 1 oder n und m 1 sind; E eine $C_1$—$C_3$-Alkylenbrücke bedeutet und X für Sauerstoff oder Schwefel steht.

6. Verbindungen der Formel I nach Anspruch 5, dadurch gekennzeichnet, dass $R_1$ $CF_3$ und $R_2$ $NO_2$ darstellen; $R_4$ für Wasserstoff steht und $R_5$ Halogen, $C_1$—$C_6$-Alkyl, $C_1$—$C_8$-Alkoxy, $C_1$—$C_3$-Alkylthio, durch Halogen oder $C_1$—$C_4$-Alkoxy substituiertes $C_1$—$C_6$-Alkyl oder $C_1$—$C_4$-Alkoxy; $R_6$ Halogen, $C_1$—$C_6$-Alkyl, $C_1$—$C_8$-Alkoxy, $C_1$—$C_3$-Alkylthio, $C_3$—$C_6$-Alkenyl, durch Halogen oder $C_1$—$C_4$-Alkoxy substituiertes $C_1$—$C_6$-Alkyl oder $C_1$—$C_4$-Alkoxy; und $R_7$ Halogen, $C_1$—$C_6$-Alkyl, $C_1$—$C_8$-Alkoxy, $C_1$—$C_3$-Alkylthio, $C_2$—$C_5$-Alkenyl-$CH_2$-oxy, $C_2$—$C_5$-Alkinyl-$CH_2$-oxy, durch Halogen substituiertes $C_1$—$C_6$-Alkyl oder durch Halogen substituiertes $C_1$—$C_4$-Alkoxy bedeuten.

7. Eine Verbindung der Formel I nach Anspruch 1, ausgewählt aus der Reihe:
N-(2'-Trifluormethyl-4',6'-dinitrophenyl)-2-amino-4-chlor-6-difluorpyrimidin;
N-(2'-Trifluormethyl-4',6'-dinitrophenyl)-2-amino-4,6-dichlormethoxy-pyrimidin;
N-(2'-Trifluormethyl-4',6'-dinitrophenyl)-2-amino-4,5,6-trichlor-pyrimidin;
N-(2'-Trifluormethyl-4',6'-dinitrophenyl)-2-amino-4,5-dichlor-6-methoxy-pyrimidin;
N-(2'-Trifluormethyl-4',6'-dinitrophenyl)-2-amino-4-chloro-5-brom-6-propin(2)yloxy-pyrimidin;
N-(2'-Trifluormethyl-4',6'-dinitrophenyl)-2-amino-4-chlor-5-brom-6-propen(2)yloxy-pyrimidin;
N-(2'-Trifluormethyl-4',6'-dinitrophenyl)-2-amino-4,5-dichlor-6-(2,2,2-trifluorethoxy)-pyrimidin.

8. Verfahren zur Herstellung von Verbindungen der Formel I, dadurch gekennzeichnet, dass man eine Verbindung der Formel II

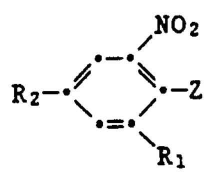

(II)

in Gegenwart einer Base mit einem Pyrimidin-Derivat der Formel III

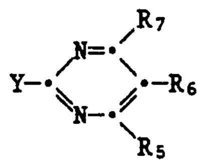

(III)

zu einer Verbindung der Formel I'

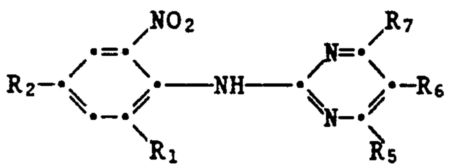

(I')

umsetzt, und letztere zur Herstellung N-acylierter Derivate mit einem reaktionsfähigen Derivat der Carbonsäure IV

$R_4COOH$ (IV)

N-acyliert, wobei die Substituenten $R_1$ bis $R_7$ die unter Formel I angegebenen Bedeutungen haben und Z und Y für $NH_2$ oder Halogen stehen, wobei in dem Fall, in dem Z für Halogen steht, Y $NH_2$ bedeutet und in dem Fall in dem Z für $NH_2$ steht, Y Halogen bedeutet.

9. Mittel zur Bekämpfung oder Verhütung eines Befalls durch Schädlinge, dadurch gekennzeichnet, dass es als aktive Komponente mindestens eine Verbindung der Formel I gemäss Anspruch 1 enthält.

10. Mittel zur Bekämpfung von Mikroorganismen gemäss Anspruch 9, dadurch gekennzeichnet, dass es als aktive Komponente mindestens eine Verbindung der Formel I gemäss einem der Ansprüche 2 bis 7 enthält.

11. Mittel nach Anspruch 9, dadurch gekennzeichnet, dass es 0,1 bis 99 Gew.-% eines Wirkstoff der Formel I, 99,9 bis 1 Gew.-% eines festen oder flüssigen Zusatzstoffes und 0 bis 25 Gew.-% eines Tensides enthält.

12. Mittel nach Anspruch 11, dadurch gekennzeichnet, dass es 0,1 bis 95 Gew.-% eines Wirkstoffs der Formel I, 99,8 bis 5 Gew.-% eines festen oder flüssigen Zusatzstoffes und 0 bis 25 Gew.-% eines Tensides enthält.

13. Verfahren zur Bekämpfung oder Verhütung eines Befalls von Kulturpflanzen durch phytopathogene Schädlinge, dadurch gekennzeichnet, dass man eine gemäss Anspruch 1 definierte Verbindung der Formel I auf die Pflanze oder deren Standort appliziert.

14. Verfahren gemäss Anspruch 13, dadurch gekennzeichnet, dass es sich bei den Schädlingen um Mikroorganismen handelt.

15. Verfahren gemäss Anspruch 14, dadurch gekennzeichnet, dass es sich bei den Mikroorganismen um phytopathogene Pilze handelt.

16. Verfahren gemäss Anspruch 15 gegen Pilze aus den Klassen Ascomycetes, Basidiomycetes oder Fungi imperfecti.

**Patentansprüche für den Vertragsstaat: AT**

1. Verfahren zur Herstellung von Verbindungen der allgemeinen Formel I

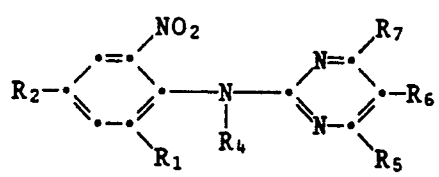

(I)

worin

$R_1$ und $R_2$ für $NO_2$ oder $CF_3$ stehen, mit der Massgabe, dass nur entweder $R_1$ oder $R_2$ für $NO_2$ stehen kann;

$R_4$ für Wasserstoff oder die Gruppe —C(O)R' steht, wobei R' unsubstituiertes oder durch Halogen oder $C_1$—$C_3$-Alkoxy substituiertes $C_1$—$C_4$-Alkyl bedeutet;

$R_5$ für Halogen, Cyano, $C_1$—$C_{12}$-Alkyl, $C_1$—$C_8$-Alkylthio, $C_1$—$C_6$-Alkylsulfonyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom

unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt oder n und n und m 0 sind; E eine $C_1$—$C_3$-Alkylenbrücke bedeutet und X für Sauerstoff oder Schwefel steht;

$R_6$ für Halogen, Nitro, Cyano, $C_1$—$C_{12}$-Alkyl, $C_1$—$C_8$-Alkylthio, $C_1$—$C_6$-Alkylsulfonyl, $C_3$—$C_6$-Alkenyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, $C_1$—$C_3$-Alkyl, $C_1$—$C_3$-Alkoxy der Nitro substituiertes Phenyl darstellt und n und m 0 sind; E eine $C_1$—$C_3$-Alkylenbrücke bedeutet und X für Sauerstoff oder Schwefel steht, und $R_6$ zusätzlich Cyclohexyloxy darstellt.

$R_7$ für Halogen, Rhodano, $C_1$—$C_{12}$-Alkyl, $C_1$—$C_8$-Alkylthio, $C_1$—$C_6$-Alkylsulfonyl, $C_1$—$C_6$-Alkylsulfoxyl, $C_2$—$C_5$-Alkenyl-$CH_2$-oxy, $C_2$—$C_5$-Alkinyl-$CH_2$-oxy, durch Halogen substituiertes $C_1$—$C_8$-Alkyl, unsubstituiertes oder durch Halogen substituiertes $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 0 und m 1 oder n und m 1 sind; E eine $C_1$—$C_3$-Alkylenbrücke bedeutet und X für Sauerstoff oder Schwefel steht; und

$R_6$ und/oder einer der Reste $R_5$ oder $R_7$ auch für Wasserstoff stehen können, dadurch gekennzeichnet, dass man eine Verbindung der Formel II

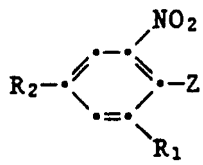

(II)

in Gegenwart einer Base mit einem Pyrimidin-Derivat der Formel III

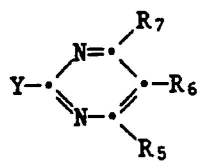

(III)

zu einer Verbindung der Formel I'

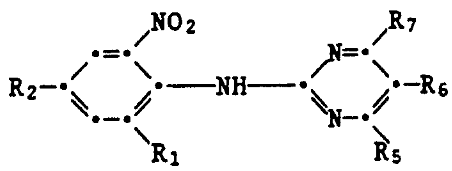

(I')

umsetzt, und letztere zur Herstellung N-acylierter Derivate mit einem reaktionsfähigen Derivat der Carbonsäure IV

$$R_4COOH \qquad (IV)$$

N-acyliert, wobei die Substituenten $R_1$ bis $R_7$ die unter Formel I angegebenen Bedeutungen haben und Z und Y für $NH_2$ oder Halogen stehen, wobei in dem Fall, in dem Z für Halogen steht, Y $NH_2$ bedeutet und in dem Fall in dem Z für $NH_2$ steht, Y Halogen bedeutet.

2. Verfahren nach Anspruch 1 zur Herstellung von Verbindungen der Formel I, worin $R_1$, $R_2$ und $R_4$ die unter Formel angegebenen Bedeutungen haben

$R_5$ für Halogen, Cyano, $C_1$—$C_6$-Alkyl, $C_1$—$C_3$-Alkylthio, $C_1$—$C_3$-Alkylsulfonyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E für eine Methylenbrücke steht und X für Sauerstoff oder Schwefel bedeutet;

$R_6$ für Halogen, Nitro, Cyano, $C_1$—$C_6$-Alkyl, $C_1$—$C_3$-Alkylthio, $C_1$—$C_3$-Alkylsulfonyl, $C_3$—$C_4$-Alkenyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, Methyl, Methoxy oder Nitro substituiertes Phenyl darstellt und n und m 0 sind; E für eine Methylenbrücke steht und X Sauerstoff oder Schwefel bedeutet;

$R_7$ für Halogen, $C_1$—$C_6$-Alkyl, $C_1$—$C_3$-Alkylthio, $C_1$—$C_3$-Akylsulfonyl, $C_1$—$C_3$-Alkylsulfoxyl, durch Halogen substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 0 und m 1 oder n und m 1 sind; E für eine Methylenbrücke steht und X Sauerstoff oder Schwefel bedeutet; und

$R_6$ und/oder einer der Reste $R_5$ oder $R_7$ auch für Wasserstoff stehen können.

3. Verfahren nach Anspruch 2 zur Herstellung von Verbindungen der Formel, worin $R_1$ und $R_2$ die unter Formel I angegebenen Bedeutungen haben;

$R_4$ Wasserstoff darstellt; und

$R_5$ für Halogen, Cyano, $C_1$—$C_6$-Alkyl, $C_1$—$C_3$-Alkylthio, $C_1$—$C_3$-Alkylsulfonyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E für eine Methylenbrücke steht und X Sauerstoff oder Schwefel bedeutet;

$R_6$ für Halogen, Nitro, Cyano, $C_1$—$C_6$-Alkyl, $C_1$—$C_3$-Alkylthio, $C_1$—$C_3$-Alkylsulfonyl, $C_3$—$C_4$-Alkenyl, durch Halogen und/oder $C_1$—$C_3$-Alkoxy substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, Methyl, Methoxy oder Nitro substituiertes Phenyl darstellt und n und m 0 sind; E für eine Methylenbrücke steht und X Sauerstoff oder Schwefel bedeutet;

$R_7$ für Halogen, $C_1$—$C_6$-Alkyl, $C_1$—$C_3$-Alkylthio, $C_1$—$C_3$-Alkylsulfonyl, $C_1$—$C_3$-Alkylsulfoxyl, durch Halogen substituiertes $C_1$—$C_3$-Alkyl, $C_1$—$C_6$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt un n und m 0 oder n 0 oder m 1 oder n und m 1 sind; E für eine Methylenbrücke steht und X Sauerstoff oder Schwefel bedeutet; und

$R_6$ und/oder einer der Reste $R_5$ oder $R_7$ auch für Wasserstoff stehen können.

4. Verfahren nach Anspruch 1 zur Herstellung von Verbindungen der Formel I, worin $R_1$ für $CF_3$ steht; $R_2$ für $NO_2$ steht; $R_4$ und $R_6$ Wasserstoff bedeuten; $R_5$ für Fluor, Chlor, Brom, $C_1$—$C_3$-Alkyl, $C_1$—$C_3$-Haloalkyl oder $C_1$—$C_3$-Alkoxy steht; und $R_7$ für Fluor, Chlor, Brom, $C_1$—$C_3$-Alkyl, $C_1$—$C_3$-Haloalkyl, $C_1$—$C_3$-Alkoxy oder $C_1$—$C_3$-Haloalkoxy steht.

5. Verfahren nach Anspruch 1 zur Herstellung von Verbindungen der Formel I, worin $R_1$ $CF_3$ und $R_2$ $NO_2$ darstellen; für Wasserstoff steht;

$R_5$ für Halogen, Cyano, $C_1$—$C_{12}$-Alkyl, $C_1$—$C_8$-Alkylthio, $C_1$—$C_6$-Alkylsulfonyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n und m 1 sind, oder worin Q ein durch $CF_3$ substituiertes Phenyl darstellt und n und m 0 sind; E eine $C_1$—$C_3$-Alkylenbrücke bedeutet und X für Sauerstoff oder Schwefel steht;

$R_6$ für Halogen, Nitro, Cyano, $C_1$—$C_{12}$-Alkyl, $C_1$—$C_8$-Alkylthio, $C_1$—$C_6$-Alkylsulfonyl, $C_3$—$C_6$-Alkenyl, durch Halogen und/oder $C_1$—$C_4$-Alkoxy substituiertes $C_1$—$C_8$-Alkyl, $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 1 und m 0 oder n 0 und m 1 sind, oder worin Q ein durch Halogen, $C_1$—$C_3$-Alkyl, $C_1$—$C_3$-Alkoxy oder Nitro substituiertes Phenyl darstellt und n und m 0 sind; E eine $C_1$—$C_3$-Alkylenbrücke bedeutet und X für Sauerstoff oder Schwefel steht, und $R_6$ zusätzlich Cyclohexyloxy darstellt.

$R_7$ für Halogen, Rhodano, $C_1$—$C_{12}$-Alkyl, $C_1$—$C_8$-Alkylthio, $C_1$—$C_6$-Alkylsulfonyl, $C_1$—$C_6$-Alkylsulfoxyl, $C_2$—$C_5$-Alkenyl-$CH_2$-oxy, $C_2$—$C_5$-Alkinyl-$CH_2$-oxy, durch Halogen substituiertes $C_1$—$C_8$-Alkyl, unsubstituiertes oder durch Halogen substituiertes $C_1$—$C_8$-Alkoxy, dessen Alkylteil durch ein Sauerstoffatom unterbrochen sein kann, oder für die Gruppe Q—$(E)_n$—$(X)_m$ steht, worin Q unsubstituiertes Phenyl darstellt und n und m 0 oder n 0 und m 1 oder n und m 1 sind; E eine $C_1$—$C_3$-Alkylenbrücke bedeutet und X für Sauerstoff oder Schwefel steht.

6. Verfahren nach Anspruch 5 zur Herstellung von Verbindungen der Formel I, worin $R_1$ $CF_3$ und $R_2$ $NO_2$ darstellen; $R_4$ für Wasserstoff steht und $R_5$ Halogen, $C_1$—$C_6$-Alkyl, $C_1$—$C_8$-Alkoxy, $C_1$—$C_3$-Alkylthio, durch Halogen oder $C_1$—$C_4$-Alkoxy substituiertes $C_1$—$C_6$-Alkyl oder $C_1$—$C_4$-Alkoxy; $R_6$ Halogen, $C_1$—$C_6$-Alkyl, $C_1$—$C_8$-Alkoxy, $C_1$—$C_3$-Alkylthio, $C_3$—$C_6$-Alkenyl, durch Halogen oder $C_1$—$C_4$-Alkoxy substituiertes $C_1$—$C_6$-Alkyl oder $C_1$—$C_4$-Alkoxy; und $R_7$ Halogen, $C_1$—$C_6$-Alkyl, $C_1$—$C_8$-Alkoxy, $C_1$—$C_3$-Alkylthio, $C_2$—$C_5$-Alkenyl-$CH_2$-oxy, $C_2$—$C_5$-Alkinyl-$CH_2$-oxy, durch Halogen substituiertes $C_1$—$C_6$-Alkyl oder durch Halogen substituiertes $C_1$—$C_4$-Alkoxy bedeuten.

7. Verfahren nach Anspruch 1 zur Herstellung von Verbindungen der Formel I nach Anspruch 1, ausgewählt aus der Reihe:

N-(2'-Trifluormethyl-4',6'-dinitrophenyl)-2-amino-4-chlor-6-difluormethylpyrimidin;

N-(2'-Trifluormethyl-4',6'-dinitrophenyl)-2-amino-4,6-dichlor-pyrimidin;

N-(2'-Trifluormethyl-4',6'-dinitrophenyl)-2-amino-4,5,6-trichlor-pyrimidin;

N-(2'-Trifluormethyl-4',6'-dinitrophenyl)-2-amino-4,5-dichlor-6-methoxy-pyrimidin;

N-(2'-Trifluormethyl-4',6'-dinitrophenyl)-2-amino-4-chloro-5-brom-6-propin(2)yloxy-pyrimidin;

N-(2'-Trifluormethyl-4',6'-dinitrophenyl)-2-amino-4-chlor-5-brom-6-propen(2)yloxy-pyrimidin;
N-(2'-Trifluormethyl-4',6'-dinitrophenyl)-2-amino-4,5-dichlor-6-(2,2,2-trifluorethoxy)-pyrimidin.

8. Mittel zur Bekämpfung oder Verhütung eines Befalls durch Schädlinge, dadurch gekennzeichnet, dass es als aktive Komponente mindestens eine Verbindung der Formel I gemäss Anspruch 1 enthält.

9. Mittel zur Bekämpfung von Mikroorganismen gemäss Anspruch 9, dadurch gekennzeichnet, dass es als aktive Komponente mindestens eine Verbindung der Formel I gemäss einem der Ansprüche 2 bis 7 enthält.

10. Mittel nach Anspruch 8, dadurch gekennzeichnet, dass es 0,1 bis 99 Gew.-% eines Wirkstoff der Formel I, 99,9 bis 1 Gew.-% eines festen oder flüssigen Zusatzstoffes und 0 bis 25 Gew.-% eines Tensides enthält.

11. Mittel nach Anspruch 10, dadurch gekennzeichnet, dass es 0,1 bis 95 Gew.-% eines Wirkstoffs der Formel I, 99,8 bis 5 Gew.-% eines festen oder flüssigen Zusatzstoffes und 0 bis 25 Gew.-% eines Tensides enthält.

12. Verfahren zur Bekämpfung oder Verhütung eines Befalls von Kulturpflanzen durch phytopathogene Schädlinge, dadurch gekennzeichnet, dass man eine gemäss Anspruch 1 definierte Verbindung der Formel I auf die Pflanze oder deren Standort appliziert.

13. Verfahren gemäss Anspruch 12 zur Bekämpfung und/oder präventiven Verhütung eines Befalls von Mikroorganismen.

14. Verfahren gemäss Anspruch 13, dadurch gekennzeichnet, dass es sich bei den Mikroorganismen um phytopathogene Pilze handelt.

15. Verfahren gemäss Anspruch 14 gegen Pilze aus den Klassen Ascomycetes, Basidiomycetes oder Fungi imperfecti.

**Revendications pour les Etats contractants: BE CH DE FR GB IT LI LU NL SE**

1. Composés de formule générale I

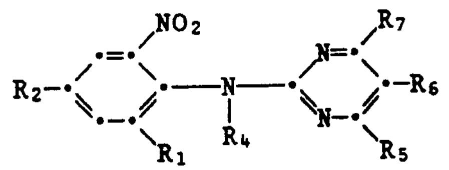

(I)

dans laquelle

$R_1$ et $R_2$ représentent $NO_2$ ou $CF_3$ sous réserve qu'un seul de ces symboles peut représenter $NO_2$;

$R_4$ représente l'hydrogène ou le groupe —C(O)R' dans lequel R' représente un groupe alkyle en $C_1$—$C_4$ non substitué ou substitué par des halogènes ou des groupes alcoxy en $C_1$—$C_3$;

$R_5$ représente un halogène, un groupe cyano, alkyle en $C_1$—$C_{12}$, alkylthio en $C_1$—$C_8$, alkylsulfonyle en $C_1$—$C_6$, alkyle en $C_1$—$C_8$ substitué par des halogènes et/ou des groupes alcoxy en $C_1$—$C_4$, alcoxy en $C_1$—$C_8$ dont la partie alkyle peut être interrompue par un atome d'oxygène, ou bien le groupe Q—$(E)_n$—$(X)_m$ dans lequel Q représente un groupe phényle non substitué et n et m sont égaux à 0 ou bien n est égal à 1 et m à 0 ou bien n et m sont égaux à 1, ou dans lequel Q représente un groupe phényle substitué par $CF_3$ et n et m sont égaux à 0; E représente un pont alkylène en $C_1$—$C_3$ et X l'oxygène ou le soufre;

$R_6$ représente un halogène, un groupe nitro, cyano, alkyle en $C_1$—$C_{12}$, alkylthio en $C_1$—$C_8$, alkylsulfonyle en $C_1$—$C_6$, alcényle en $C_3$—$C_6$, alkyle en $C_1$—$C_8$ substitué par des halogènes et/ou des groupes alkyle en $C_1$—$C_4$, alcoxy en $C_1$—$C_8$ dont la partie alkyle peut être interrompue par un atome d'oxygène, ou le groupe Q—$(E)_n$—$(X)_m$ dans lequel Q représente un groupe phényle non substitué et n et m sont égaux à ou un n à 1 et m à 0 ou n à 0 et m à 1, ou bien dans lequel Q représente un groupe phényle substitué par des halogènes, des groupes alkyle en $C_1$—$C_3$, alcoxy en $C_1$—$C_3$ ou nitro et n et m sont égaux à 0; E représente un pont alkylène en $C_1$—$C_3$ et X l'oxygène ou le soufre, $R_6$ pouvant en outre représenter un groupe cyclohexyloxy;

$R_7$ représente un halogène, un groupe thiocyano, alkyle en $C_1$—$C_{12}$, alkylthio en $C_1$—$C_8$, alkylsulfonyle en $C_1$—$C_6$, alkylsulfoxyle en $C_1$—$C_6$, (alcényle en $C_2$—$C_5$)-$CH_2$-oxy, (alcynyle en $C_2$—$C_5$)-$CH_2$-oxy, alkyle en $C_1$—$C_8$ substitué par des halogènes, alcoxy en $C_1$—$C_8$ non substitué ou substitué par des halogènes et dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 0 et m à 1 ou n et m à 1; E représente un pont alkylène en $C_1$—$C_3$ et X l'oxygène ou le soufre; et

$R_6$ et/ou l'un des symboles $R_5$ et $R_7$ peuvent également représenter l'hydrogène.

2. Composés de formule I selon la revendication 1, caractérisés en ce que $R_1$, $R_2$ et $R_4$ ont les significations indiquées en référence à la formule I;

$R_5$ représente un halogène, un groupe cyano, alkyle en $C_1$—$C_6$, alkylthio en $C_1$—$C_3$, alkylsulfonyle en $C_1$—$C_3$, alkyle en $C_1$—$C_3$ substitué par des halogènes et/ou des groupes alcoxy en $C_1$—$C_3$, alcoxy en $C_1$—$C_6$ dont la partie alkyle peut être interrompue par un atome d'oxygène, ou le groupe Q—$(E)_n$—$(X)_m$ dans lequel

Q représente un groupe phényle non substitué et n et m sont égaux à 0 ou n à 1 et m à 0 ou n et m à 1, ou bien dans lequel Q représente un groupe phényle substitué par $CF_3$ et n et m sont égaux à 0; E représente un pont méthylène et X l'oxygène ou le soufre;

$R_6$ représente un halogène, un groupe nitro, cyano, alkyle en $C_1$—$C_6$, alkylthio en $C_1$—$C_3$, alkylsulfonyle en $C_1$—$C_3$, alcényle en $C_3$—$C_4$, alkyle en $C_1$—$C_3$ substitué par des halogènes et/ou des groupes alcoxy en $C_1$—$C_3$, alcoxy en $C_1$—$C_6$ dont la partie alkyle peut être interrompue par un atome d'oxygène ou le groupe Q—$(E)_n$—$(X)_m$ dans lequel Q représente un groupe phényle non substitué et n et m sont égaux à 0 ou n à 1 et m à 0 ou n à 0 et m à 1, ou dans lequel Q représente un groupe phényle substitué par des halogènes, des groupes méthyle, méthoxy ou nitro et n et m sont égaux à 0; E représente un pont méthylène et X l'oxygène ou le soufre;

$R_7$ représente un halogène, un groupe alkyle en $C_1$—$C_6$, alkylthio en $C_1$—$C_3$, alkylsulfonyle en $C_1$—$C_3$, alkylsulfoxyle en $C_1$—$C_3$, alkyle en $C_1$—$C_3$ substitué par des halogènes, alcoxy en $C_1$—$C_6$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 0 et m à 1 ou n et m à 1; E représente un pont méthylène et X l'oxygène ou le soufre; et

$R_6$ et/ou l'un des symboles $R_5$ et $R_7$ peuvent également représenter l'hydrogène.

3. Composés de formule I selon la revendication 2, caractérisés en ce que $R_1$ et $R_2$ ont les significations indiquées en référence à la formule I;

$R_4$ représente l'hydrogène; et

$R_5$ représente un halogène, un groupe cyano, alkyle en $C_1$—$C_6$, alkylthio en $C_1$—$C_3$, alkylsulfonyle en $C_1$—$C_3$, alkyle en $C_1$—$C_3$ substitué par des halogènes et/ou des groupes alcoxy en $C_1$—$C_3$, alcoxy en $C_1$—$C_6$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 1 et m à 0 ou n et m à 1, ou dans lequel Q représente un groupe phényle substitué par $CF_3$ et n et m sont égaux à 0; E représente un pont méthylène et X l'oxygène ou le soufre;

$R_6$ représente un halogène, un groupe nitro, cyano, alkyle en $C_1$—$C_6$, alkylthio en $C_1$—$C_3$, alkylsulfonyle en $C_1$—$C_3$, alcényle en $C_3$—$C_4$, alkyle en $C_1$—$C_3$ substitué par des halogènes et/ou des groupes alcoxy en $C_1$—$C_3$, alcoxy en $C_1$—$C_6$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 1 et m à 0 ou n à 0 et m à 1, ou dans lequel Q représente un groupe phényle substitué par des halogènes, des groupes méthyle, méthoxy ou nitro et n et m sont égaux à 0; E représente un pont méthylène et X l'oxygène ou le soufre;

$R_7$ représente un halogène, un groupe alkyle en $C_1$—$C_6$, alkylthio en $C_1$—$C_3$, alkylsulfonyle en $C_1$—$C_3$, alkylsulfoxyle en $C_1$—$C_3$, alkyle en $C_1$—$C_3$ substitué par des halogènes, alcoxy en $C_1$—$C_6$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 0 et m à 1 ou n et m à 1; E représente un pont méthylène et X l'oxygène ou le soufre; et

$R_6$ et/ou l'un des symboles $R_5$ et $R_7$ peuvent également représenter l'hydrogène.

4. Composés de formule I selon la revendication 1, caractérisés en ce que $R_1$ représente $CF_3$; $R_2$ représente $NO_2$; $R_4$ et $R_6$ représentent l'hydrogène; $R_5$ représente le fluor, le chlore, le brome, un groupe alkyle en $C_1$—$C_3$, halogénoalkyle en $C_1$—$C_3$ ou alcoxy en $C_1$—$C_3$; et $R_7$ représente le fluor, le chlore, le brome, un groupe alkyle en $C_1$—$C_3$, halogénoalkyle en $C_1$—$C_3$, alcoxy en $C_1$—$C_3$ ou halogénoalcoxy en $C_1$—$C_3$.

5. Composés de formule I selon la revendication 1, caractérisés en ce que:

$R_1$ représente $CF_3$ et $R_2$ $NO_2$; $R_4$ représente l'hydrogène;

$R_5$ représente un halogène, un groupe cyano, alkyle en $C_1$—$C_{12}$, alkylthio en $C_1$—$C_8$, alkylsulfonyle en $C_1$—$C_6$ alkyle en $C_1$—$C_8$ substitué par des halogènes et/ou des groupes alcoxy en $C_1$—$C_4$, alcoxy en $C_1$—$C_8$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 1 et m à 0 ou n et m à 1, ou bien dans lequel Q représente un groupe phényle substitué par $CF_3$ et n et m sont égaux à 0; E représente un pont alkylène en $C_1$—$C_3$ et X l'oxygène ou le soufre;

$R_6$ représente un halogène, un groupe nitro, cyano, alkyle en $C_1$—$C_{12}$, alkylthio en $C_1$—$C_8$, alkylsulfonyle en $C_1$—$C_6$, alcényle en $C_3$—$C_6$, alkyle en $C_1$—$C_8$ substitué par des halogènes et/ou des groupes alcoxy en $C_1$—$C_4$, alcoxy en $C_1$—$C_8$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 1 et m à 0 ou n à 0 et m à 1, ou dans lequel Q représente un groupe phényle substitué par des halogènes, des groupes alkyle en $C_1$—$C_3$, alcoxy en $C_1$—$C_3$ ou nitro et n et m sont égaux à 0; E représente un pont alkylène en $C_1$—$C_3$ et X l'oxygène ou le soufre, $R_6$ pouvant en outre représenter un groupe cyclohexyloxy,

$R_7$ représente un halogène, un groupe thiocyano, alkyle en $C_1$—$C_{12}$, alkylthio en $C_1$—$C_8$, alkylsulfonyle en $C_1$—$C_6$, alkylsulfoxyle en $C_1$—$C_6$, (alcényle en $C_2$—$C_5$)-$CH_2$-oxy, (alcynyle en $C_2$—$C_5$)-$CH_2$-oxy, alkyle en $C_1$—$C_8$ substitué par des halogènes, alcoxy en $C_1$—$C_8$ non substitué ou substitué par des halogènes et dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 0 et m à 1 ou n et m à 1; E représente un pont alkylène en $C_1$—$C_3$ et X l'oxygène ou le soufre.

6. Composés de formule I selon la revendication 5, caractérisés en ce que $R_1$ représente $CF_3$ et $R_2$ $NO_2$; $R_4$ représente l'hydrogène et $R_5$ un halogène, un groupe alkyle en $C_1$—$C_6$, alcoxy en $C_1$—$C_8$, alkylthio en $C_1$—$C_3$, alkyle en $C_1$—$C_6$ substitué par des halogènes ou des groupes alcoxy en $C_1$—$C_4$, ou alcoxy en $C_1$—$C_4$; $R_6$ représente un halogène, un groupe alkyle en $C_1$—$C_6$, alcoxy en $C_1$—$C_8$, alkylthio en $C_1$—$C_3$, alcényle en $C_3$—$C_6$, alkyle en $C_1$—$C_6$ substitué par des halogènes ou des groupes alcoxy en $C_1$—$C_4$, ou alcoxy en $C_1$—$C_4$; et $R_7$ représente un halogène, un groupe alkyle en $C_1$—$C_6$, alcoxy en $C_1$—$C_8$, alkylthio en $C_1$—$C_3$, (alcényle en $C_2$—$C_5$)-$CH_2$-oxy, (alcynyle en $C_2$—$C_5$)-$CH_2$-oxy, alkyle en $C_1$—$C_6$ substitué par des halogènes ou alcoxy en $C_1$—$C_4$ substitué par des halogènes.

7. Un composé de formule I selon la revendication 1, choisi parmi les suivants:
N-(2'-trifluorométhyl-4',6'-dinitrophényl)-2-amino-4-chloro-6-difluorométhoxy-pyrimidine;
N-(2'-trifluorométhyl-4',6'-dinitrophényl)-2-amino-4,6-dichloropyrimidine;
N-(2'-trifluorométhyl-4',6'-dinitrophényl)-2-amino-4,5,6-trichloropyrimidine;
N-(2'-trifluorométhyl-4',6'-dinitrophényl)-2-amino-4,5-dichloro-6-méthoxypyrimidine;
N-(2'-trifluorométhyl-4',6'-dinitrophényl)-2-amino-4-chloro-5-bromo-6-propyne-2-yloxy-pyrimidine;
N-(2'-trifluorométhyl-4',6'-dinitrophényl)-2-amino-4-chloro-5-bromo-6-propène-2-yloxy-pyrimidine;
N-(2'-trifluorométhyl-4',6'-dinitrophényl)-2-amino-4,5-dichloro-6-(2,2,2-trifluoréthoxy)-pyrimidine.

8. Procédé de préparation des composés de formule I, caractérisé en ce que l'on fait réagir un composé de formule II

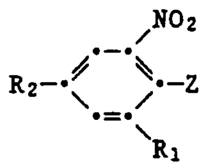

(II)

en présence d'une base, avec un dérivé de la pyrimidine répondant à la formule III

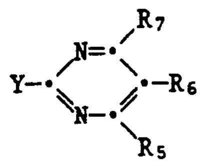

(III)

ce qui donne un composé de formule I'

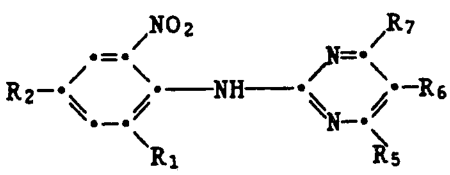

(I')

qu'on soumet, pour la préparation des dérivés N-acylés, à N-acylation à l'aide d'un dérivé réactif de l'acide carboxylique IV

$$R_4COOH \qquad (IV)$$

les symboles $R_1$ à $R_7$ ayant les significations indiquées en référence à la formule I et Z et Y représentant $NH_2$ ou des halogènes, sous réserve que lorsque Z représente un halogène, Y représente $NH_2$ et lorsque Z représente $NH_2$, Y représente un halogène.

9. Produit pour combattre ou prévenir une attaque par des parasites, caractérisé en ce qu'il contient en tant que composant actif au moins un composé de formule I selon la revendication 1.

10. Produit pour combattre les microorganismes selon la revendication 9, caractérisé en ce qu'il contient en tant que composant actif au moins un composé de formule I selon l'une des revendications 2 à 7.

11. Produit selon la revendication 9, caractérisé en ce qu'il contient de 0,1 à 99% en poids d'une substance active de formule I, de 99,9 à 1% en poids d'un additif solide ou liquide et de 0 à 25% en poids d'un agent tensioactif.

12. Produit selon la revendication 11, caractérisé en ce qu'il contient de 0,1 à 95% en poids d'une substance active de formule I, de 99,8 à 5% en poids d'un additif solide ou liquide et de 0 à 25% en poids d'un agent tensioactif.

13. Procédé pour combattre ou prévenir une attaque de végétaux cultivés par des parasites

phytopathogènes, caractérisé en ce que l'on applique un composé de formule I de la revendication 1 sur la plante ou son habitat.

14. Procédé selon la revendication 13, caractérisé en ce que les parasites sont des microorganismes.

15. Procédé selon la revendication 14, caractérisé en ce que les microorganismes sont des mycètes phytopathogènes.

16. Procédé selon la revendication 15, contre des mycètes appartenant aux classes des ascomycètes, des basidiomycètes ou des Fungi imperfecti.

**Revendications pour l'Etat contractant: AT**

1. Procédé de préparation de composés de formule générale

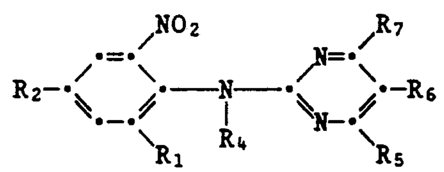

(I)

dans laquelle

$R_1$ et $R_2$ représentent $NO_2$ ou $CF_3$ sous réserve qu'un seul de ces symboles peut représenter $NO_2$;

$R_4$ représente l'hydrogène ou le groupe —C(O)R' dans lequel R' représente un groupe alkyle en $C_1$—$C_4$ non substitué ou substitué par des halogènes ou des groupes alcoxy en $C_1$—$C_3$;

$R_5$ représente un halogène, un groupe cyano, alkyle en $C_1$—$C_{12}$, alkylthio en $C_1$—$C_8$, alkylsulfonyle en $C_1$—$C_6$, alkyle en $C_1$—$C_8$ substitué par des halogènes et/ou des groupes alcoxy en $C_1$—$C_4$, alcoxy en $C_1$—$C_8$ dont la partie alkyle peut être interrompue par un atome d'oxygène, ou bien le groupe $Q—(E)_n—(X)_m$ dans lequel Q représente un groupe phényle non substitué et n et m sont égaux à 0 ou bien n est égal à 1 et m à 0 ou bien n et m sont égaux à 1, ou dans lequel Q représente un groupe phényle substitué par $CF_3$ et n et m sont égaux à 0; E représente un pont alkylène en $C_1$—$C_3$ et X l'oxygène ou le soufre;

$R_6$ représente un halogène, un groupe nitro, cyano, alkyle en $C_1$—$C_{12}$, alkylthio en $C_1$—$C_8$, alkylsulfonyle en $C_1$—$C_6$, alcényle en $C_3$—$C_6$, alkyle en $C_1$—$C_8$ substitué par des halogènes et/ou des groupes alkyle en $C_1$—$C_4$, alcoxy en $C_1$—$C_8$ dont la partie alkyle peut être interrompue par un atome d'oxygène, ou le groupe $Q—(E)_n—(X)_m$ dans lequel Q représente un groupe phényle non substitué et n et m sont égaux à ou un n à 1 et m à 0 ou n à 0 et m à 1, ou bien dans lequel Q représente un groupe phényle substitué par des halogènes, des groupes alkyle en $C_1$—$C_3$, alcoxy en $C_1$—$C_3$ ou nitro et n et m sont égaux à 0; E représente un pont alkylène en $C_1$—$C_3$ et X l'oxygène ou le soufre, $R_6$ pouvant en outre représenter un groupe cyclohexyloxy;

$R_7$ représente un halogène, un groupe thiocyano, alkyle en $C_1$—$C_{12}$, alkylthio en $C_1$—$C_8$, alkylsulfonyle en $C_1$—$C_6$, alkylsulfoxyle en $C_1$—$C_6$, (alcényle en $C_2$—$C_5$)-$CH_2$-oxy, (alcynyle en $C_2$—$C_5$)-$CH_2$-oxy, alkyle en $C_1$—$C_8$ substitué par des halogènes, alcoxy en $C_1$—$C_8$ non substitué ou substitué par des halogènes et dont la partie alkyle peut être interrompue par un atome d'oxygène, ou le groupe $Q—(E)_n—(X)_m$ dans lequel Q représente un groupe phényle non substitué et n et m sont égaux à 0 ou n à 0 et m à 1 ou n et m à 1; E représente un pont alkylène en $C_1$—$C_3$ et X l'oxygène ou le soufre; et

$R_6$ et/ou l'un des symboles $R_5$ et $R_7$ peuvent également représenter l'hydrogène;

caractérisé en ce que l'on fait réagir un composé de formule II

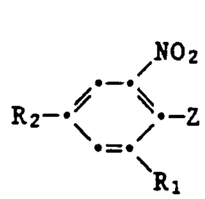

(II)

en présence d'une base, avec un dérivé de la pyrimidine répondant à la formule III

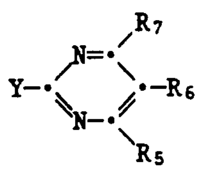

(III)

ce qui donne un composé de formule I'

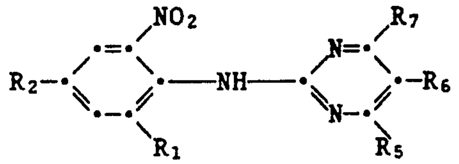

(I')

qu'on soumet, pour la préparation des dérivés N-acylés, à n-acylation à l'aide d'un dérivé réactif de l'acide carboxylique IV

$$R_4COOH \quad (IV)$$

les symboles $R_1$ à $R_7$ ayant les significations indiquées en référence à la formule I et Z et Y représentant $NH_2$ ou des halogènes, sous réserve que lorsque Z représente un halogène, Y représente $NH_2$ et lorsque Z représente $NH_2$, Y représente un halogène.

2. Procédé selon la revendication 1, pour la préparation de composés de formule I dans laquelle $R_1$, $R_2$ et $R_4$ ont les significations indiquées en référence à la formule I;

$R_5$ représente un halogène, un groupe cyano, alkyle en $C_1$—$C_6$, alkylthio en $C_1$—$C_3$, alkylsulfonyle en $C_1$—$C_3$, alkyle en $C_1$—$C_3$ substitué par des halogènes et/ou des groupes alcoxy en $C_1$—$C_3$, alcoxy en $C_1$—$C_6$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 1 et m à 0 ou n et m à 1, ou bien dans lequel Q représente un groupe phényle substitué par $CF_3$ et n et m sont égaux à 0; E représente un pont méthylène et X l'oxygène ou le soufre;

$R_6$ représente un halogène, un groupe nitro, cyano, alkyle en $C_1$—$C_6$, alkylthio en $C_1$—$C_3$, alkylsulfonyle en $C_1$—$C_3$, alcényle en $C_3$—$C_4$, alkyle en $C_1$—$C_3$ substitué par des halogènes et/ou des groupes alcoxy en $C_1$—$C_3$, alcoxy en $C_1$—$C_6$ dont la partie alkyle peut être interrompue par un atome d'oxygène ou le groupe Q—$(E)_n$—$(X)_m$ dans lequel Q représente un groupe phényle non substitué et n et m sont égaux à 0 ou n à 1 et m à 0 ou n à 0 et m à 1, ou dans lequel Q représente un groupe phényle substitué par des halogènes, des groupes méthyle, méthoxy ou nitro et n et m sont égaux à 0; E représente un pont méthylène et X l'oxygène ou le soufre;

$R_7$ représente un halogène, un groupe alkyle en $C_1$—$C_6$, alkylthio en $C_1$—$C_3$, alkylsulfonyle en $C_1$—$C_3$, alkylsulfoxyle en $C_1$—$C_3$, alkyle en $C_1$—$C_3$ substitué par des halogènes, alcoxy en $C_1$—$C_6$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 0 et m à 1 ou n et m à 1; E représente un pont méthylène et X l'oxygène ou le soufre; et

$R_6$ et/ou l'un des symboles $R_5$ et $R_7$ peuvent également représenter l'hydrogène.

3. Procédé selon la revendication 2, pour la préparation de composés de formule I dans laquelle $R_1$ et $R_2$ ont les significations indiquées en référence à la formule I;

$R_4$ représente l'hydrogène; et

$R_5$ représente un halogène, un groupe cyano, alkyle en $C_1$—$C_6$, alkylthio en $C_1$—$C_3$, alkylsulfonyle en $C_1$—$C_3$, alkyle en $C_1$—$C_3$ substitué par des halogènes et/ou des groupes alcoxy en $C_1$—$C_3$, alcoxy en $C_1$—$C_6$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 1 et m à 0 ou n et m à 1, ou dans lequel Q représente un groupe phényle substitué par $CF_3$ et n et m sont égaux à 0; E représente un pont méthylène et X l'oxygène ou le soufre;

$R_6$ représente un halogène, un groupe nitro, cyano, alkyle en $C_1$—$C_6$, alkylthio en $C_1$—$C_3$, alkylsulfonyle en $C_1$—$C_3$, alcényle en $C_3$—$C_4$, alkyle en $C_1$—$C_3$ substitué par des halogènes et/ou des groupes alcoxy en $C_1$—$C_3$, alcoxy en $C_1$—$C_6$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 1 et m à 0 ou n à 0 et m à 1, ou dans lequel Q représente un groupe phényle substitué par des halogènes, des groupes méthyle, méthoxy ou nitro et n et m sont égaux à 0; E représente un pont méthylène et X l'oxygène ou le soufre;

$R_7$ représente un halogène, un groupe alkyle en $C_1$—$C_6$, alkylthio en $C_1$—$C_3$, alkylsulfonyle en $C_1$—$C_3$, alkylsulfoxyle en $C_1$—$C_3$, alkyle en $C_1$—$C_3$ substitué par des halogènes, alcoxy en $C_1$—$C_6$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 0 et m à 1 ou n et m à 1; E représente un pont méthylène et X l'oxygène ou le soufre; et

$R_6$ et/ou l'un des symboles $R_5$ et $R_7$ peuvent également représenter l'hydrogène.

4. Procédé selon la revendication 1, pour la préparation de composés de formule I dans laquelle $R_1$ représente $CF_3$; $R_2$ représente $NO_2$; $R_4$ et $R_6$ représentent l'hydrogène; $R_5$ représente le fluor, le chlor, le brome, un groupe alkyle en $C_1$—$C_3$, halogénoalkyle en $C_1$—$C_3$ ou alcoxy en $C_1$—$C_3$; et $R_7$ représente le fluor, le chlore, le brome, un groupe alkyle en $C_1$—$C_3$, halogénoalkyle en $C_1$—$C_3$, alcoxy en $C_1$—$C_3$ ou halogénoalcoxy en $C_1$—$C_3$.

5. Procédé selon la revendication 1, pour la préparation de composés de formule I dans laquelle: $R_1$ représente $CF_3$ et $R_2$ $NO_2$; $R_4$ représente l'hydrogène;

$R_5$ représente un halogène, un groupe cyano, alkyle en $C_1$—$C_{12}$, alkylthio en $C_1$—$C_8$, alkylsulfonyle en $C_1$—$C_6$ alkyle en $C_1$—$C_8$ substitué par des halogènes et/ou des groupes alcoxy en $C_1$—$C_4$, alcoxy en $C_1$—$C_8$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 1 et m à 0 ou n et m à 1, ou bien dans lequel Q représente un groupe phényle substitué par $CF_3$ et n et m sont égaux à 0; E représente un pont alkylène en $C_1$—$C_3$ et X l'oxygène ou le soufre;

$R_6$ représente un halogène, un groupe nitro, cyano, alkyle en $C_1$—$C_{12}$, alkylthio en $C_1$—$C_8$, alkylsulfonyle en $C_1$—$C_6$, alcényle en $C_3$—$C_6$, alkyle en $C_1$—$C_8$ substitué par des halogènes et/ou des groupes alcoxy en $C_1$—$C_4$, alcoxy en $C_1$—$C_8$ dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 1 et m à 0 ou n à 0 et m à 1, ou dans lequel Q représente un groupe phényle substitué par des halogènes, des groupes alkyle en $C_1$—$C_3$, alcoxy en $C_1$—$C_3$ ou nitro et n et m sont égaux à 0; E représente un pont alkylène en $C_1$—$C_4$ et X l'oxygène ou le soufre, $R_6$ pouvant en outre représenter un groupe cyclohexyloxy,

$R_7$ représente un halogène, un groupe thiocyano, alkyle en $C_1$—$C_{12}$, alkylthio en $C_1$—$C_8$, alkylsulfonyle en $C_1$—$C_6$, alkylsulfoxyle en $C_1$—$C_6$, (alcényle en $C_2$—$C_5$)-$CH_2$-oxy, (alcynyle en $C_2$—$C_5$)-$CH_2$-oxy, alkyle en $C_1$—$C_8$ substitué par des halogènes, alcoxy en $C_1$—$C_8$ non substitué ou substitué par des halogènes et dont la partie alkyle peut être interrompue par un atome d'oxygène, ou le groupe Q—$(E)_n$—$(X)_m$ dans lequel Q représente un groupe phényle non substitué et n et m sont égaux à 0 ou n à 0 et m à 1 ou n et m à 1; E représente un pont alkylène en $C_1$—$C_3$ et X l'oxygène ou le soufre.

6. Procédé selon la revendication 5, pour la préparation de composés de formule I dans laquelle $R_1$ représente $CF_3$ et $R_2$ $NO_2$; $R_4$ représente l'hydrogène et $R_5$ un halogène, un groupe alkyle en $C_1$—$C_6$, alcoxy en $C_1$—$C_8$, alkylthio en $C_1$—$C_3$, alkyle en $C_1$—$C_6$ substitué par des halogènes ou des groupes alcoxy en $C_1$—$C_4$, ou alcoxy en $C_1$—$C_4$; $R_6$ représente un halogène, un groupe alkyle en $C_1$—$C_6$, alcoxy en $C_1$—$C_8$, alkylthio en $C_1$—$C_3$, alcényle en $C_3$—$C_6$, alkyle en $C_1$—$C_6$ substitué par des halogènes ou des groupes alcoxy en $C_1$—$C_4$, ou alcoxy en $C_1$—$C_4$; et $R_7$ représente un halogène, un groupe alkyle en $C_1$—$C_6$, alcoxy en $C_1$—$C_8$, alkylthio en $C_1$—$C_3$, (alcényle en $C_2$—$C_5$)-$CH_2$-oxy, (alcynyle en $C_2$—$C_5$)-$CH_2$-oxy, alkyle en $C_1$—$C_6$ substitué par des halogènes ou alcoxy en $C_1$—$C_4$ substitué par des halogènes.

7. Procédé selon la revendication 1, pour la préparation de composés de formule I selon la revendication 1, choisis parmi les suivants:

N-(2'-trifluorométhyl-4',6'-dinitrophényl)-2-amino-4-chloro-6-difluorométhoxy-pyrimidine;
N-(2'-trifluorométhyl-4',6'-dinitrophényl)-2-amino-4,6-dichloropyrimidine;
N-(2'-trifluorométhyl-4',6'-dinitrophényl)-2-amino-4,5,6-trichloropyrimidine;
N-(2'-trifluorométhyl-4',6'-dinitrophényl)-2-amino-4,5-dichloro-6-méthoxypyrimidine;
N-(2'-trifluorométhyl-4',6'-dinitrophényl)-2-amino-4-chloro-5-bromo-6-propyne-2-yloxy-pyrimidine;
N-(2'-trifluorométhyl-4',6'-dinitrophényl)-2-amino-4-chloro-5-bromo-6-propène-2-yloxy-pyrimidine;
N-(2'-trifluorométhyl-4',6'-dinitrophényl)-2-amino-4,5-dichloro-6-(2,2,2-trifluoréthoxy)-pyrimidine.

8. Produit pour combattre ou prévenir une attaque par des parasites, caractérisé en ce qu'il contient en tant que composant actif au moins un composé de formule I selon la revendication 1.

9. Produit pour combattre les microorganismes seon la revendication 8, caractérisé en ce qu'il contient en tant que composant actif au moins un composé de formule I selon l'une des revendications 2 à 7.

10. Produit selon la revendication 8, caractérisé en ce qu'il contient de 0,1 à 99% en poids d'une substance active de formule I, de 99,9 à 1% en poids d'un additif solide ou liquide et de 0 à 25% en poids d'un agent tensioactif.

11. Produit selon la revendication 10, caractérisé en ce qu'il contient de 0,1 à 95% en poids d'une substance active de formule I, de 99,8 à 5% en poids d'un additif solide ou liquide et de 0 à 25% en poids d'un agent tensioactif.

12. Procédé pour combattre ou prévenir une attaque de végétaux cultivés par des parasites phytopathogènes, caractérisé en ce que l'on applique sur la plante ou son habitat un composé de formule I défini dans la revendication 1.

13. Procédé selon la revendication 12, pour combattre et/ou prévenir une attaque par des microorganismes.

14. Procédé selon la revendication 13, caractérisé en ce que les microorganismes sont des mycètes phytopathogènes.

15. Procédé selon la revendication 14, contre des mycètes des classes des ascomycètes, des basidiomycètes et Fungi imperfecti.

**Claims for the Contracting States: BE CH DE FR GB IT LI LU NL SE**

1. A compound of the general formula I

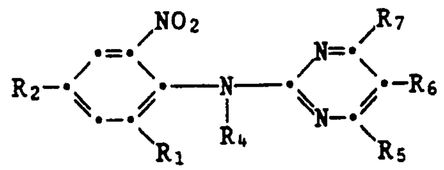

(I)

in which

$R_1$ and $R_2$ are $NO_2$ or $CF_3$, with the proviso that only either $R_1$ or $R_2$ can be $NO_2$;

$R_4$ is hydrogen or the —C(O)R′ group, in which R′ is $C_1$—$C_4$alkyl which is unsubstituted or substituted by halogen or $C_1$—$C_3$alkoxy;

$R_5$ is halogen, cyano, $C_1$—$C_{12}$alkyl, $C_1$—$C_8$alkylthio, $C_1$—$C_6$alkylsulfonyl, $C_1$—$C_8$alkyl which is substituted by halogen and/or $C_1$—$C_4$alkoxy, or is $C_1$—$C_8$alkoxy, the alkyl moiety of which may be interrupted by an oxygen atom, or is the Q—$(E)_n$—$(X)_m$ group in which Q is unsubstituted phenyl and n and m are 0 or n is 1 and m is 0 or n and m are 1, or in which Q is phenyl which is substituted by $CF_3$ and n and m are 0; E is a $C_1$—$C_3$alkylene bridge and X is oxygen or sulfur.

$R_6$ is halogen, nitro, cyano $C_1$—$C_{12}$alkyl, $C_1$—$C_8$alkylthio, $C_1$—$C_6$alkylsulfonyl, $C_3$—$C_6$alkenyl, $C_1$—$C_8$alkyl which is substituted by halogen and/or $C_1$—$C_4$alkoxy, or is $C_1$—$C_8$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n is 0 and m is 1, or in which Q is phenyl which is substituted by halogen, $C_1$—$C_3$alkyl, $C_1$—$C_3$alkoxy or nitro and n and m are 0; E is a $C_1$—$C_3$alkylene bridge and X is oxygen or sulfur, and $R_6$ is additionally cyclohexyloxy;

$R_7$ is halogen, thiocyanato, $C_1$—$C_{12}$alkyl, $C_1$—$C_8$alkylthio, $C_1$—$C_6$alkylsulfonyl, $C_1$—$C_6$alkylsulfoxyl, $C_2$—$C_5$alkenyl-$CH_2$-oxy, $C_2$—$C_5$alkynyl-$CH_2$-oxy, $C_1$—$C_8$alkyl which is substituted by halogen, $C_1$—$C_8$alkoxy which is unsubstituted or substituted by halogen, the alkyl moiety of which may be interrupted by an oxygen atom, or is the Q—$(E)_n$—$(X)_m$ group, in which Q is unsubstituted phenyl and n and m are 0 or n is 0 and m is 1 or n and m are 1; E is a $C_1$—$C_3$alkylene bridge and X is oxygen or sulfur; and

$R_6$ and/or one of the radicals $R_5$ or $R_7$ may also be hydrogen.

2. A compound of the formula I according to claim 1, wherein $R_1$, $R_2$ and $R_4$ are as defined for formula I;

$R_5$ is halogen, cyano, $C_1$—$C_6$alkyl, $C_1$—$C_3$alkylthio, $C_1$—$C_3$alkylsulfonyl, $C_1$—$C_3$alkyl which is substituted by halogen and/or $C_1$—$C_3$alkoxy, $C_1$—$C_6$alkoxy,.the alkyl moiety of which may be interrupted by an oxygen atom, or is the Q—$(E)_n$—$(X)_m$ group, in which Q is unsubstituted phenyl and n and m are 0 or n is 1 and m is 0 or n and m are 1, or in which Q is a phenyl which is substituted by $CF_3$ and n and m are 0; E is a methylene bridge and X is oxygen or sulfur;

$R_6$ is halogen, nitro, cyano, $C_1$—$C_6$alkyl, $C_1$—$C_3$alkylthio, $C_1$—$C_3$alkylsulfonyl, $C_3$—$C_4$alkenyl, $C_1$—$C_3$alkyl which is substituted by halogen and/or $C_1$—$C_3$alkoxy, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n is 0 and m is 1, or in which Q is a phenyl which is substituted by halogen, methyl, methoxy or nitro and n and m are 0; E is a methylene bridge and X is oxygen or sulfur;

$R_7$ is halogen, $C_1$—$C_6$alkyl, $C_1$—$C_3$alkylthio, $C_1$—$C_3$alkylsulfonyl, $C_1$—$C_3$alkylsulfoxyl, $C_1$—$C_3$alkyl which is substituted by halogen, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 0 and m is 1 or n and m are 1; E is a methylene bridge and X is oxygen or sulfur; and

$R_6$ and/or one of the radicals $R_5$ or $R_7$ may also be hydrogen.

3. A compound of the formula I according to claim 2, wherein $R_1$ and $R_2$ are as defined for formula I;

$R_4$ is hydrogen; and

$R_5$ is halogen, cyano, $C_1$—$C_6$alkyl, $C_1$—$C_3$alkylthio, $C_1$—$C_3$alkylsulfonyl, $C_1$—$C_3$alkyl which is substituted by halogen and/or $C_1$—$C_3$alkoxy, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n and m are 1, or in which Q is a phenyl which is substituted by $CF_3$ and n and m are 0; E is a methylene bridge and X is oxygen or sulfur;

$R_6$ is halogen, nitro, cyano, $C_1$—$C_6$alkyl, $C_1$—$C_3$alkylthio, $C_1$—$C_3$alkylsulfonyl, $C_3$—$C_4$alkenyl, $C_1$—$C_3$alkyl which is substituted by halogen and/or $C_1$—$C_3$alkoxy, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n is 0 and m is 1, or in which Q is a phenyl which is substituted by halogen, methyl, methoxy or nitro and n and m are 0; E is a methylene bridge and X is oxygen or sulfur;

$R_7$ is halogen, $C_1$—$C_6$alkyl, $C_1$—$C_3$alkylthio, $C_1$—$C_3$alkylsulfonyl, $C_1$—$C_3$alkylsulfoxyl, $C_1$—$C_3$alkyl which is substituted by halogen, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 0 and m is 1 or n and m are 1; E is a methylene bridge and X is oxygen or sulfur; and

$R_6$ and/or one of the radicals $R_5$ or $R_7$ may also be hydrogen.

4. A compound of the formula I according to claim 1, wherein $R_1$ is $CF_3$; $R_2$ is $NO_2$; $R_4$ and $R_6$ are hydrogen; $R_5$ is fluorine, chlorine, bromine, $C_1$—$C_3$alkyl, $C_1$—$C_3$haloalkyl or $C_1$—$C_3$alkoxy; and $R_7$ is fluorine, chlorine, bromine, $C_1$—$C_3$alkyl, $C_1$—$C_3$haloalkyl, $C_1$—$C_3$alkoxy or $C_1$—$C_3$haloalkoxy.

5. A compound of the formula I according to claim 1, wherein $R_1$ is $CF_3$ and $R_2$ is $NO_2$; $R_4$ is hydrogen;

$R_5$ is halogen, cyano, $C_1$—$C_{12}$alkyl, $C_1$—$C_8$alkylthio, $C_1$—$C_6$alkylsulfonyl, $C_1$—$C_8$alkyl which is substituted by halogen and/or $C_1$—$C_4$alkoxy, or is $C_1$—$C_8$alkoxy, the alkyl moiety of which may be interrupted by an oxygen atom, or is a Q—$(E)_n$—$(X)_m$ group, in which Q is unsubstituted phenyl and n and m are 0 and n is 1 and m is 0 or n and m are 1, or in which Q is phenyl which is substituted by $CF_3$ and n and m are 0; E is a $C_1$—$C_3$alkylene bridge and X is oxygen or sulfur;

$R_6$ is halogen, nitro, cyano, $C_1$—$C_{12}$alkyl, $C_1$—$C_8$alkylthio, $C_1$—$C_6$alkylsulfonyl, $C_3$—$C_6$alkenyl, $C_1$—$C_8$alkyl which is substituted by halogen and/or $C_1$—$C_4$alkoxy, or is $C_1$—$C_8$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n is 0 and m is 1, or in which Q is phenyl which is substituted by halogen, $C_1$—$C_3$alkyl, $C_1$—$C_3$alkoxy or nitro and n and m are 0; E is a $C_1$—$C_3$alkylene bridge and X is oxygen or sulfur, and $R_6$ is additionally cyclohexyloxy;

$R_7$ is halogen, thiocyanato, $C_1$—$C_{12}$alkyl, $C_1$—$C_8$alkylthio, $C_1$—$C_6$alkylsulfonyl, $C_1$—$C_6$alkylsulfoxy, $C_2$—$C_5$alkenyl-$CH_2$-oxy, $C_2$—$C_5$alkynyl-$CH_2$-oxy, $C_1$—$C_8$alkyl which is substituted by halogen, $C_1$—$C_8$alkoxy which is unsubstituted or substituted by halogen, the alkyl moiety of which may be interrupted by an oxygen atom, or is the Q—$(E)_n$—$(X)_m$ group, in which Q is unsubstituted phenyl and n and m are 0 or n is 0 and m is 1 or n and m are 1; E is a $C_1$—$C_3$alkylene bridge and X is oxygen or sulfur.

6. A compound of the formula I according to claim 5, wherein $R_1$ is $CF_3$ and $R_2$ is $NO_2$; $R_4$ is hydrogen and $R_5$ is halogen, $C_1$—$C_6$alkyl, $C_1$—$C_8$alkoxy, $C_1$—$C_3$alkylthio, or is $C_1$—$C_6$alkyl or $C_1$—$C_4$alkoxy, each of which is substituted by halogen or $C_1$—$C_4$alkoxy; $R_6$ is halogen, $C_1$—$C_6$alkyl, $C_1$—$C_8$alkoxy, $C_1$—$C_3$alkylthio, $C_3$—$C_6$alkenyl, or is $C_1$—$C_6$alkyl or $C_1$—$C_4$alkoxy each of which is substituted by halogen or $C_1$—$C_4$alkoxy; and $R_7$ is halogen, $C_1$—$C_6$alkyl, $C_1$—$C_8$alkoxy, $C_1$—$C_3$alkylthio, $C_2$—$C_5$alkenyl-$CH_2$-oxy, $C_2$—$C_5$-alkynyl-$CH_2$-oxy, $C_1$—$C_6$alkyl which is substituted by halogen or $C_1$—$C_4$alkoxy which is substituted by halogen.

7. A compound of the formula I according to claim 1, selected from the series:

N-(2'-trifluoromethyl-4',6'-dinitrophenyl)-2-amino-4-chloro-6-difluoromethoxypyrimidine;
N-(2'-trifluoromethyl-4',6'-dinitrophenyl)-2-amino-4,6-dichloropyrimidine;
N-(2'-trifluoromethyl-4',6'-dinitrophenyl)-2-amino-4,5,6-trichloropyrimidine;
N-(2'-trifluoromethyl-4',6'-dinitrophenyl)-2-amino-4,5-dichloro-6-methoxy-pyrimidine;
N-(2'-trifluoromethyl-4',6'-dinitrophenyl)-2-amino-4-chloro-5-bromo-6-propyn-2-yloxy-pyrimidine;
N-(2'-trifluoromethyl-4',6'-dinitrophenyl)-2-amino-4-chloro-5-bromo-6-propen-2-yloxy-pyrimidine;
N-(2'-trifluoromethyl-4',6'-dinitrophenyl)-2-amino-4,5-dichloro-6-(2,2,2-trifluoroethoxy)-pyrimidine.

8. A process for the preparation of a compound of formula I, which process comprises reacting a compound of the formula II

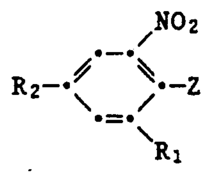

(II)

with a pyrimidine derivative of the formula III

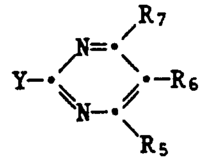

(III)

in the presence of a base, to give a compound of the formula I'

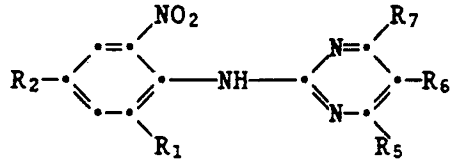

(I')

and to prepare an N-acylated derivative, N-acylating the compound of formula I' with a reactive derivative of the carboxylic acid of the formula IV

$R_4COOH$ (IV)

the substituents $R_1$ to $R_7$ being as defined for formula I and Z and Y being $NH_2$ or halogen, with the proviso that, if Z is halogen, Y is $NH_2$ and, if Z is $NH_2$, Y is halogen.

9. A composition for controlling or preventing attack by pests, which contains as active component at least one compound of the formula I according to claim 1.

10. A composition for controlling microorganisms according to claim 9, which contains as active component at least one compound of the formula I according to any one of claims 2 to 7.

11. A composition according to claim 9, which contains 0.1 to 99% by weight of active compound of the formula I, 99.9 to 1% by weight of a solid or liquid adjuvant, and 0 to 25% by weight of a surfactant.

12. A composition according to claim 11, which contains 0.1 to 95% by weight of an active compound of the formula I, 99.8 to 5% by weight of a solid or liquid adjuvant, and 0 to 25% by weight of a surfactant.

13. A method of controlling or of preventing attack of cultivated plants by phytopathogenic pests, which comprises applying to said plants or to the locus thereof a compound of the formula I as defined in claim 1.

14. A method according to claim 13, wherein the pests are microorganisms.

15. A method according to claim 14, wherein the microorganisms are phytopathogenic fungi.

16. A method according to claim 15, wherein the phytopathogenic fungi are fungi of the classes Ascomycetes, Basidiomycetes or Fungi imperfecti.

**Claims for the Contracting State: AT**

1. A process for the preparation of a compound of the general formula I

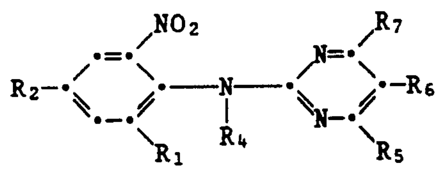

(I)

in which

$R_1$ and $R_2$ are $NO_2$ or $CF_3$, with the proviso that only either $R_1$ or $R_2$ can be $NO_2$;

$R_4$ is hydrogen or the —C(O)R′ group, in which R′ is $C_1$—$C_4$alkyl which is unsubstituted or substituted by halogen or $C_1$—$C_3$alkoxy;

$R_5$ is halogen, cyano, $C_1$—$C_{12}$alkyl, $C_1$—$C_8$alkylthio, $C_1$—$C_6$alkylsulfonyl, $C_1$—$C_8$alkyl which is substituted by halogen and/or $C_1$—$C_4$alkoxy, or is $C_1$—$C_8$alkoxy, the alkyl moiety of which may be interrupted by an oxygen atom, or is the Q—$(E)_n$—$(X)_m$ group in which Q is unsubstituted phenyl and n and m are 0 or n is 1 and m is 0 or n and m are 1, or in which Q is phenyl which is substituted by $CF_3$ and n and m are 0; E is a $C_1$—$C_3$alkylene bridge and X is oxygen or sulfur.

$R_6$ is halogen, nitro, cyano $C_1$—$C_{12}$alkyl, $C_1$—$C_8$alkylthio, $C_1$—$C_6$alkylsulfonyl, $C_3$—$C_6$alkenyl, $C_1$—$C_8$alkyl which is substituted by halogen and/or $C_1$—$C_4$alkoxy, or is $C_1$—$C_8$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n is 0 and m is 1, or in which Q is phenyl which is substituted by halogen, $C_1$—$C_3$alkyl, $C_1$—$C_3$alkoxy or nitro and n and m are 0; E is a $C_1$—$C_3$alkylene bridge and X is oxygen or sulfur, and $R_6$ is additionally cyclohexyloxy;

$R_7$ is halogen, thiocyanato, $C_1$—$C_{12}$alkyl, $C_1$—$C_8$alkylthio, $C_1$—$C_6$alkylsulfonyl, $C_1$—$C_6$alkylsulfoxyl, $C_2$—$C_5$alkenyl-$CH_2$-oxy, $C_2$—$C_5$alkynyl-$CH_2$-oxy, $C_1$—$C_8$alkyl which is substituted by halogen, $C_1$—$C_8$alkoxy which is unsubstituted or substituted by halogen, the alkyl moiety of which may be interrupted by an oxygen atom, or is the Q—$(E)_n$—$(X)_m$ group, in which Q is unsubstituted phenyl and n and m are 0 or n is 0 and m is 1 or n and m are 1; E is a $C_1$—$C_3$alkylene bridge and X is oxygen or sulfur; and

$R_6$ and/or one of the radicals $R_5$ or $R_7$ may also be hydrogen,

which process comprises reacting a compound of the formula II

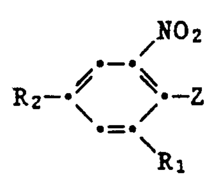

(II)

with a pyrimidine derivative of the formula III

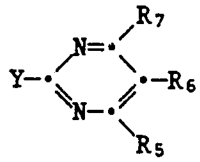

(III)

in the presence of a base, to give a compound of the formula I'

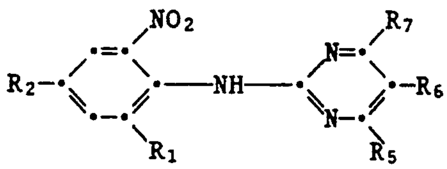

(I')

and to prepare an N-acylated derivative, N-acylating the compound of formula I' with a reactive derivative of the carboxylic acid of the formula IV

$$R_4COOH \quad (IV)$$

the substituents $R_1$ to $R_7$ being as defined for formula I and Z and Y being $NH_2$ or halogen, with the proviso that, if Z is halogen, Y is $NH_2$ and, if Z is $NH_2$, Y is halogen.

2. A process according to claim 1 for the preparation of a compound of the formula I, wherein $R_1$, $R_2$ and $R_4$ are as defined for formula I;

$R_5$ is halogen, cyano, $C_1$—$C_6$alkyl, $C_1$—$C_3$alkylthio, $C_1$—$C_3$alkylsulfonyl, $C_1$—$C_3$alkyl which is substituted by halogen and/or $C_1$—$C_3$alkoxy, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n and m are 1, or in which Q is a phenyl which is substituted by $CF_3$ and n and m are 0; E is a methylene bridge and X is oxygen or sulfur;

$R_6$ is halogen, nitro, cyano, $C_1$—$C_6$alkyl, $C_1$—$C_3$alkylthio, $C_1$—$C_3$alkylsulfonyl, $C_3$—$C_4$alkenyl, $C_1$—$C_3$alkyl which is substituted by halogen and/or $C_1$—$C_3$alkoxy, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n is 0 and m is 1, or in which Q is a phenyl which is substituted by halogen, methyl, methoxy or nitro and n and m are 0; E is a methylene bridge and X is oxygen or sulfur;

$R_7$ is halogen, $C_1$—$C_6$alkyl, $C_1$—$C_3$alkylthio, $C_1$—$C_3$alkylsulfonyl, $C_1$—$C_3$alkylsulfoxyl, $C_1$—$C_3$alkyl which is substituted by halogen, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 0 and m is 1 or n and m are 1; E is a methylene bridge and X is oxygen or sulfur; and

$R_6$ and/or one of the radicals $R_5$ or $R_7$ may also be hydrogen.

3. A process according to claim 2 for the preparation of a compound of the formula I, wherein $R_1$ and $R_2$ are as defined for formula I;

$R_4$ is hydrogen; and

$R_5$ is halogen, cyano, $C_1$—$C_6$alkyl, $C_1$—$C_3$alkylthio, $C_1$—$C_3$alkylsulfonyl, $C_1$—$C_3$alkyl which is substituted by halogen and/or $C_1$—$C_3$alkoxy, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n and m are 1, or in which Q is a phenyl which is substituted by $CF_3$ and n and m are 0; E is a methylene bridge and X is oxygen or sulfur;

$R_6$ is halogen, nitro, cyano, $C_1$—$C_6$alkyl, $C_1$—$C_3$alkylthio, $C_1$—$C_3$alkylsulfonyl, $C_3$—$C_4$alkenyl, $C_1$—$C_3$alkyl which is substituted by halogen and/or $C_1$—$C_3$alkoxy, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n is 0 and m is 1, or in which Q is a phenyl which is substituted by halogen, methyl, methoxy or nitro and n and m are 0; E is a methylene bridge and X is oxygen or sulfur;

$R_7$ is halogen, $C_1$—$C_6$alkyl, $C_1$—$C_3$alkylthio, $C_1$—$C_3$alkylsulfonyl, $C_1$—$C_3$alkylsulfoxyl, $C_1$—$C_3$alkyl which is substituted by halogen, $C_1$—$C_6$alkoxy, the alkyl moiety of which may be interrupted by an oxygen atom, or is the Q—$(E)_n$—$(X)_m$ group, in which Q is unsubstituted phenyl and n and m are 0 or n is 0 and m is 1 or n and m are 1; E is a methylene bridge and X is oxygen or sulfur; and

$R_6$ and/or one of the radicals $R_5$ or $R_7$ may also be hydrogen.

4. A process according to claim 1 for the preparation of a compound of formula I, wherein $R_1$ is $CF_3$; $R_2$ is $NO_2$; $R_4$ and $R_6$ are hydrogen; $R_5$ is fluorine, chlorine, bromine, $C_1$—$C_3$alkyl, $C_1$—$C_3$haloalkyl or $C_1$—$C_3$alkoxy; and $R_7$ is fluorine, chlorine, bromine, $C_1$—$C_3$alkyl, $C_1$—$C_3$haloalkyl, $C_1$—$C_3$alkoxy or $C_1$—$C_3$haloalkoxy.

5. A process according to claim 1 for the preparation of a compound of the formula I, wherein $R_1$ is $CF_3$ and $R_2$ is $NO_2$; $R_4$ is hydrogen;

$R_5$ is halogen, cyano, $C_1$—$C_{12}$alkyl, $C_1$—$C_8$alkylthio, $C_1$—$C_6$alkylsulfonyl, $C_1$—$C_8$alkyl which is substituted by halogen and/or $C_1$—$C_4$alkoxy, or is $C_1$—$C_8$alkoxy, the alkyl moiety of which may be interrupted by an oxygen atom, or is a Q—$(E)_n$—$(X)_m$ group, in which Q is unsubstituted phenyl and n and m are 0 and n is 1 and m is 0 or n and m are 1, or in which Q is phenyl which is substituted by $CF_3$ and n and m are 0; E is a $C_1$—$C_3$alkylene bridge and X is oxygen or sulfur;

$R_6$ is halogen, nitro, cyano, $C_1$—$C_{12}$alkyl, $C_1$—$C_8$alkylthio, $C_1$—$C_6$alkylsulfonyl, $C_3$—$C_6$alkenyl, $C_1$—$C_8$alkyl which is substituted by halogen and/or $C_1$—$C_4$alkoxy, or is $C_1$—$C_8$alkoxy, the alkyl moiety of which may be interrupted by an oxygen atom, or is the Q—$(E)_n$—$(X)_m$ group, in which Q is unsubstituted phenyl and n and m are 0 or n is 1 and m is 0 or n is 0 and m is 1, or in which Q is phenyl which is substituted by halogen, $C_1$—$C_3$alkyl, $C_1$—$C_3$alkoxy or nitro and n and m are 0; E is a $C_1$—$C_3$alkylene bridge and X is oxygen or sulfur, and $R_6$ is additionally cyclohexyloxy;

$R_7$ is halogen, thiocyanato, $C_1$—$C_{12}$alkyl, $C_1$—$C_8$alkylthio, $C_1$—$C_6$alkylsulfonyl, $C_1$—$C_6$alkylsulfoxy, $C_2$—$C_5$alkenyl-$CH_2$-oxy, $C_2$—$C_5$alkynyl-$CH_2$-oxy, $C_1$—$C_8$alkyl which is substituted by halogen, $C_1$—$C_8$alkoxy which is unsubstituted or substituted by halogen, the alkyl moiety of which may be interrupted by an oxygen atom, or is the Q—$(E)_n$—$(X)_m$ group, in which Q is unsubstituted phenyl and n and m are 0 or n is 0 and m is 1 or n and m are 1; E is a $C_1$—$C_3$alkylene bridge and X is oxygen or sulfur.

6. A process according to claim 5 for the preparation of a compound of the formula I, wherein $R_1$ is $CF_3$ and $R_2$ is $NO_2$; $R_4$ is hydrogen and $R_5$ is halogen, $C_1$—$C_6$alkyl, $C_1$—$C_8$alkoxy, $C_1$—$C_3$alkylthio, or is $C_1$—$C_6$alkyl or $C_1$—$C_4$alkoxy, each of which is substituted by halogen or $C_1$—$C_4$alkoxy; $R_6$ is halogen, $C_1$—$C_6$alkyl, $C_1$—$C_8$alkoxy, $C_1$—$C_3$alkylthio, $C_3$—$C_6$alkenyl, or is $C_1$—$C_6$alkyl or $C_1$—$C_4$alkoxy each of which is substituted by halogen or $C_1$—$C_4$alkoxy; and $R_7$ is halogen, $C_1$—$C_6$alkyl, $C_1$—$C_8$alkoxy, $C_1$—$C_3$alkylthio, $C_2$—$C_5$alkenyl-$CH_2$-oxy, $C_2$—$C_5$alkynyl-$CH_2$-oxy, $C_1$—$C_6$alkyl which is substituted by halogen or $C_1$—$C_4$alkoxy which is substituted by halogen.

7. A process according to claim 1 for the preparation of a compound of the formula I according to Claim 1, selected from the series:

N-(2′-trifluoromethyl-4′,6′-dinitrophenyl)-2-amino-4-chloro-6-difluoromethoxypyrimidine;
N-(2′-trifluoromethyl-4′,6′-dinitrophenyl)-2-amino-4,6-dichloropyrimidine;
N-(2′-trifluoromethyl-4′,6′-dinitrophenyl)-2-amino-4,5,6-trichloropyrimidine;
N-(2′-trifluoromethyl-4′,6′-dinitrophenyl)-2-amino-4,5-dichloro-6-methoxy-pyrimidine;
N-(2′-trifluoromethyl-4′,6′-dinitrophenyl)-2-amino-4-chloro-5-bromo-6-propyn-2-yloxy-pyrimidine;
N-(2′-trifluoromethyl-4′,6′-dinitrophenyl)-2-amino-4-chloro-5-bromo-6-propen-2-yloxy-pyrimidine;
N-(2′-trifluoromethyl-4′,6′-dinitrophenyl)-2-amino-4,5-dichloro-6-(2,2,2-trifluoroethoxy)-pyrimidine.

8. A composition for controlling or preventing attack by pests, which contains as active component at least one compound of the formula I according to claim 1.

9. A composition for controlling microorganisms according to claim 8, which contains as active component at least one compound of the formula I according to any one of claims 2 to 7.

10. A composition according to claim 8, which contains 0.1 to 99% by weight of active compound of the formula I, 99.9 to 1% by weight of a solid or liquid adjuvant, and 0 to 25% by weight of a surfactant.

11. A composition according to claim 10, which contains 0.1 to 95% by weight of an active compound of the formula I, 99.8 to 5% by weight of a solid or liquid adjuvant, and 0 to 25% by weight of a surfactant.

12. A method of controlling or of preventing attack of cultivated plants by phytopathogenic pests, which comprises applying to said plants or to the locus thereof a compound of the formula I as defined in claim 1.

13. A method according to claim 12, for controlling and/or preventing attack by microorganisms.

14. A method according to claim 13, wherein the microorganisms are phytopathogenic fungi.

15. A method according to claim 14, wherein the phytopathogenic fungi are fungi of the classes Ascomycetes, Basidiomycetes or Fungi imperfecti.